(19) **Europäisches Patentamt / European Patent Office / Office européen des brevets**

(11) **EP 3 462 412 B1**

(12) **EUROPÄISCHE PATENTSCHRIFT**

(45) Veröffentlichungstag und Bekanntmachung des Hinweises auf die Patenterteilung:
**30.10.2019 Patentblatt 2019/44**

(51) Int Cl.:
*G06T 7/00* *(2017.01)*    *G06T 3/00* *(2006.01)*
*G06T 15/08* *(2011.01)*

(21) Anmeldenummer: **17193853.3**

(22) Anmeldetag: **28.09.2017**

(54) **BESTIMMEN EINES ZWEIDIMENSIONALEN MAMMOGRAPHIEDATENSATZES**

DETERMINING A TWO-DIMENSIONAL MAMMOGRAPHY DATA SET

DÉTERMINATION D'UN ENSEMBLE BIDIMENSIONNEL DE DONNÉES DE MAMMOGRAPHIES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**03.04.2019 Patentblatt 2019/14**

(73) Patentinhaber: **Siemens Healthcare GmbH**
**91052 Erlangen (DE)**

(72) Erfinder: **Pauly, Olivier**
**81241 München (DE)**

(56) Entgegenhaltungen:
**US-A1- 2017 011 534**

• LITJENS GEERT ET AL: "A survey on deep learning in medical image analysis", MEDICAL IMAGE ANALYSIS, Bd. 42, 26. Juli 2017 (2017-07-26), Seiten 60-88, XP085240547, ISSN: 1361-8415, DOI: 10.1016/J.MEDIA.2017.07.005
• DAVID A SCADUTO ET AL: "Determination of System Geometrical Parameters and Consistency between Scans for Contrast-Enhanced Digital Breast Tomosynthesis", 8. Juli 2012 (2012-07-08), BREAST IMAGING, SPRINGER BERLIN HEIDELBERG, BERLIN, HEIDELBERG, PAGE(S) 24 - 31, XP047013205, ISBN: 978-3-642-31270-0 * Zusammenfassung * * Abschnitt "1 Introduction" *

EP 3 462 412 B1

**Beschreibung**

Die digitale Brusttomosynthese (DBT) ist eine vielversprechende Weiterentwicklung der zweidimensionalen Mammographie, welche die derzeitige diagnostische Standardmethode ist. Indem die DBT dreidimensionale Informationen über die Brustanatomie bereitstellt, reduziert sie die Anzahl der falschpositiven der zweidimensionalen Mammographie, die durch die Überlagerung fibroglandulären Gewebes entstehen. Auf der anderen Seite erhöht eine Diagnose basierend auf der digitalen Brusttomosynthese aber die Arbeitsbelastung des diagnostizierenden Arztes, da eine Mehrzahl von Schichtaufnahmen inspiziert werden muss.

Um die Diagnose, insbesondere die Suche nach Läsionen, effizienter zu gestalten ist es vorteilhaft, neben der digitalen Brusttomographie weiterhin eine zweidimensionale Mammographie bereitzustellen. Die zweidimensionale Mammographie könnte zwar durch eine separate Aufnahme mittels ionisierender Strahlung bereitgestellt werden, dies erhöht aber die Strahlenbelastung des untersuchten Gewebes. Es ist daher vorteilhaft, einen synthetischen zweidimensionalen Mammographiedatensatz basierend auf der dreidimensionalen digitalen Brusttomosynthese bereitzustellen.

Aus der Druckschrift Felix Diekmann et al. "Thick Slices from Tomosynthesis Data Sets: Phantom Study for the Evaluation of Different Algorithms", Journal of Digital Imaging 22(5), S. 519-526 (2009) sind Ansätze zur Erstellung eines zweidimensionalen Mammographiedatensatzes, welche Teile der digitalen Brusttomosynthese summieren (beispielsweise in Form einer maximalen Intensitätsprojektion MIP, oder durch Mittelung von Schichten). Diese Verfahren sind zwar effizient, aber weisen Probleme auf, wenn verdächtige Läsionen hinter Strukturen mit hohen Intensitätswerten verborgen sind, oder wenn in der Projektion überlappende, unauffällige Strukturen falsch-positive Resultate erzeugen.

Aus der Druckschrift G. van Schie et al., "Mass detection in reconstructed digital breast tomosynthesis volumes with a computer-aided detection system trained on 2D mammograms", Medical Physics 40(4), S. 041902 (2013) ist bekannt, eine gekrümmte zweidimensionale Fläche zu erzeugen, welche die auffälligsten Läsionen der digitalen Brusttomosynthese umfasst, und diese in der Ebene darzustellen. Hierdurch entstehen aber Verzerrungen, so dass die resultierenden zweidimensionalen Bilddaten einer üblichen zweidimensionalen Mammographie unähnlich sind und eine Diagnose schwierig machen.

Aus der Druckschrift US 20170011534 A1 ist bekannt, mittels eines selbstlernenden Algorithmus Voxeln der digitalen Brusttomosynthese Relevanzwerte zuzuweisen, wobei die Relevanzwerte die Relevanz des jeweiligen Voxels für die Brustkrebsdiagnose betreffen. Basierend auf diesen Relevanzwerten wird dann ein zweidimensionaler Bilddatensatz mittels gewichteter Intensitätsprojektion (ein englischer Fachbegriff ist "weighted intensity projection") erzeugt. Auch diese Vorgehensweise ist problematisch, wenn verdächtige Läsionen hinter Strukturen mit hohen Intensitätswerten verborgen sind, oder wenn in der Projektion überlappende, unauffällige Strukturen falsch-positive Resultate erzeugen.

Es ist daher die Aufgabe der vorliegenden Erfindung, eine alternative Methode zur Erzeugung eines synthetischen zweidimensionalen Mammographiedatensatzes aus einem dreidimensionalen Mammographiedatensatz bereitzustellen, die den gesamten dreidimensionalen Mammographiedatensatz in die Erzeugung mit einbezieht.

Die Aufgabe wird gelöst durch ein Verfahren zum Bestimmen eines zweidimensionalen Mammographiedatensatzes nach Anspruch 1, durch eine Bestimmungssystem nach Anspruch 12, durch ein Computerprogrammprodukt nach Anspruch 14 sowie durch ein computerlesbares Speichermedium nach Anspruch 15.

Nachstehend wird die erfindungsgemäße Lösung der Aufgabe sowohl in Bezug auf die beanspruchten Vorrichtungen als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Die Erfindung betrifft ein Verfahren zum Bestimmen eines zweidimensionalen Mammographiedatensatzes. Das Verfahren Umfasst das Empfangen eines dreidimensionalen Mammographiedatensatzes eines Untersuchungsbereiches mittels einer Schnittstelle. Das Verfahren umfasst weiterhin das erste Bestimmen eines zweidimensionalen Mammographiedatensatzes des Untersuchungsbereiches durch Anwendung einer trainierten Generatorfunktion auf den dreidimensionalen Mammographiedatensatz mittels einer Recheneinheit, wobei die trainierte Generatorfunktion auf einem trainierten GA-Algorithmus basiert.

Der Erfinder hat erkannt, dass es durch dieses Verfahren möglich ist, synthetische zweidimensionale Mammographiedatensätze zu erzeugen, die auf dem gesamten dreidimensionalen Mammographiedatensatz basieren. Gleichzeitig werden effizient zweidimensionale Mammographiedatensätze erzeugt, die visuell ähnlich zu realen zweidimensionalen Mammographiedatensätzen sind und daher mit standardisierten Methoden befundet werden können.

Nach einem weiteren Aspekt der Erfindung umfasst der trainierte GA-Algorithmus eine generative Funktion und eine klassifizierende Funktion, wobei die trainierte Generatorfunktion mit der generativen Funktion des trainierten GA-Algorithmus identisch ist. Der Erfinder hat erkannt, dass durch diesen Aufbau des GA-Algorithmus das Training der Generatorfunktion besonders effizient und kostengünstig erfolgen kann. Insbesondere kann die generative Funktion auch als Generatorfunktion bezeichnet werden, und die klassifizierende Funktion als Klassifikatorfunktion. Handelt es sich beim GA-Algorithmus um ein GA-Netzwerk, dann kann die generative Funktion auch als generatives Teilnetzwerk und die klassifizierende Funktion auch als klassifizierendes Teilnetzwerk bezeichnet werden.

Nach einem weiteren Aspekt der Erfindung ist die trainierte Generatorfunktion ein künstliches neuronales Netzwerk. Insbesondere basiert dann die trainierte Generatorfunktion auf einem GA-Netzwerk. Der Erfinder hat erkannt, dass das Training eines künstlichen neuronalen Netzwerkes besonders effizient ist.

Nach einem weiteren Aspekt der Erfindung umfasst die trainierte Generatorfunktion eine Verkettung einer ersten Teilfunktion, einer Projektionsfunktion und einer zweiten Teilfunktion, wobei die erste Teilfunktion den dreidimensionalen Mammographiedatensatz auf einen ersten Merkmalsvektor abbildet, wobei die Projektionsfunktion den ersten Merkmalsvektor auf den zweiten Merkmalsvektor abbildet, und wobei die zweite Teilfunktion den zweiten Merkmalsvektor auf den zweidimensionalen Mammographiedatensatz abbildet. Insbesondere ist die trainierte Generatorfunktion eine Verkettung der ersten Teilfunktion, der Projektionsfunktion und der zweiten Teilfunktion. Ist die trainierte Generatorfunktion ein künstliches neuronales Netzwerk, so ist die erste Teilfunktion insbesondere ein erstes Teilnetzwerk, weiterhin ist die Projektionsfunktion insbesondere eine Projektionsschicht, und weiterhin ist die zweite Teilfunktion insbesondere ein zweites Teilnetzwerk. Erfinder hat erkannt, dass analog zu einem Autoencoder durch die Abbildung auf einen ersten und einen zweiten Merkmalsvektor die relevanten Merkmale des dreidimensionalen Mammographiedatensatzes möglichst einfach und effizient gespeichert werden können, und dass durch die Berechnung des zweidimensionalen Mammographiedatensatzes basierend auf dem ersten bzw. dem zweiten Merkmalsvektor diese relevanten Merkmale besonders einfach und effizient in den zweidimensionalen Mammographiedatensatz aufgenommen werden können.

Nach einem weiteren möglichen Aspekt der Erfindung ist das erste Teilnetzwerk und/oder das zweite Teilnetzwerk ein faltendes künstliches neuronales Netzwerk (ein englischer Fachbegriff ist "convolutional neural network"), insbesondere um ein tiefes faltendes künstliches neuronales Netzwerk (ein englischer Fachbegriff ist "deep convolutional neural network"). Insbesondere können das erste Teilnetzwerk und/oder das zweite Teilnetzwerk auch ein ausschließlich faltendes neuronales Netzwerk sein (ein englischer Fachbegriff ist "fully convolutional neural network"), also insbesondere keine vollverbundenen Schichten aufweisen. Der Erfinder hat erkannt, dass durch ein faltendes neuronales Netzwerk besonders gut dazu geeignet ist, effizient Bilddaten als Eingabewerte zu verarbeiten.

Nach einem weiteren Aspekt der Erfindung umfasst die erste Teilfunktion wenigstens einen dreidimensionalen Faltungsoperator. Wenn die erste Teilfunktion ein erstes Teilnetzwerk ist, dann ist der dreidimensionale Faltungsoperator insbesondere eine dreidimensionale Faltungsschicht. Der Erfinder hat erkannt, dass dreidimensionale Faltungsoperatoren besonders gut geeignet sind, um Merkmale von dreidimensionalen Bilddaten zu erkennen und weiterzuverarbeiten, insbesondere um aus dreidimensionalen Bilddaten einen Merkmalsvektor zu konstruieren.

Nach einem weiteren Aspekt der Erfindung umfasst die zweite Teilfunktion wenigstens einen zweidimensionalen Faltungsoperator. Wenn die zweite Teilfunktion ein zweites Teilnetzwerk ist, dann ist der zweidimensionale Faltungsoperator insbesondere eine zweidimensionale Faltungsschicht. Der Erfinder hat erkannt, dass zweidimensionale Faltungsoperatoren besonders gut geeignet sind, um Merkmale von zweidimensionalen Bilddaten zu speichern und weiterzuverarbeiten, insbesondere um aus einem Merkmalsvektor einen zweidimensionalen Bilddatensatz zu konstruieren.

Nach einem weiteren Aspekt der Erfindung ist die Projektionsfunktion ein dreidimensionaler Faltungsoperator, wobei der zweidimensionale Mammographiedatensatz bezüglich einer ersten Richtung und bezüglich einer zweiten Richtung ausgedehnt ist, wobei der dreidimensionale Mammographiedatensatz weiterhin bezüglich einer dritten Richtung ausgedehnt ist, und wobei die Ausdehnung des Faltungskerns des dreidimensionalen Faltungsoperators bezüglich der dritten Richtung auf der Ausdehnung des dreidimensionalen Mammographiedatensatzes bezüglich der dritten Richtung basiert. Hierbei wird die Ausdehnung des Faltungskerns und des dreidimensionalen Mammographiedatensatzes insbesondere in Einheiten von Pixeln gemessen. Insbesondere ist jede der Richtungen jeweils parallel zu einer Kante eines Voxels des Faltungskerns und/oder des dreidimensionalen Mammographiedatensatzes. Der Erfinder hat erkannt, dass sich durch diese Wahl des Faltungskerns der erste Merkmalsvektor des dreidimensionalen Mammographiedatensatzes einfach und genau auf den zweiten Merkmalsvektor des zweidimensionalen Mammographiedatensatzes abbilden lässt.

Nach einem weiteren möglichen Aspekt der Erfindung umfasst die trainierte Generatorfunktion eine erste Teilfunktion, wobei die erste Teilfunktion den dreidimensionalen Mammographiedatensatz auf einen ersten Merkmalsvektor abbildet, und wobei die erste Teilfunktion wenigstens einen dreidimensionalen Faltungsoperator umfasst. Insbesondere ist die erste Teilfunktion ein erstes Teilnetzwerk und der dreidimensionale Faltungsoperator eine dreidimensionale Faltungsschicht. Der Erfinder hat erkannt, dass dreidimensionale Faltungsoperatoren besonders gut geeignet sind, um Merkmale von dreidimensionalen Bilddaten zu erkennen und weiterzuverarbeiten, insbesondere um aus dreidimensionalen Bilddaten einen Merkmalsvektor zu konstruieren.

Nach einem weiteren möglichen Aspekt der Erfindung umfasst die trainierte Generatorfunktion eine zweite Teilfunktion, wobei die zweite Teilfunktion einen zweiten Merkmalsvektor auf den zweidimensionalen Mammographiedatensatz abbildet, und wobei die zweite Teilfunktion wenigstens einen zweidimensionalen Faltungsoperator umfasst. Insbesondere ist die zweite Teilfunktion ein zweite Teilnetzwerk und der zweidimensionale Faltungsoperator eine zweidimensionale Faltungsschicht. Der Erfinder hat erkannt, dass zweidimensionale Faltungsblöcke besonders gut geeignet sind, um Merkmale von zweidimensionalen Bilddaten zu speichern und weiterzuverarbeiten, insbesondere um aus einem Merkmalsvektor einen zweidimensionalen Bilddatensatz zu konstruieren.

Nach einem weiteren möglichen Aspekt der Erfindung umfasst die trainierte Generatorfunktion eine Projektionsfunktion, wobei die Projektionsfunktion den ersten Merkmalsvektor auf den zweiten Merkmalsvektor abbildet, wobei Projektionsfunktion ein dreidimensionale Faltungsoperator ist, wobei der zweidimensionale Mammographiedatensatz bezüglich einer ersten Richtung und bezüglich einer zweiten Richtung ausgedehnt ist, wobei der dreidimensionale Mammographiedatensatz weiterhin bezüglich einer dritten Richtung ausgedehnt ist, und wobei die Ausdehnung des Faltungskerns des dreidimensionalen Faltungsoperators bezüglich der dritten Richtung auf der Ausdehnung des dreidimensionalen Mammographiedatensatzes bezüglich der dritten Richtung basiert. Insbesondere ist jede der Richtungen jeweils parallel zu einer Kante eines Voxels des Faltungskerns und/oder des dreidimensionalen Mammographiedatensatzes. Der Erfinder hat erkannt, dass sich durch diese Wahl des Faltungskerns der erste Merkmalsvektor des dreidimensionalen Mammographiedatensatzes einfach und genau auf den zweiten Merkmalsvektor des zweidimensionalen Mammographiedatensatzes abbilden lässt.

Nach einem weiteren Aspekt der Erfindung ist der zweidimensionale Mammographiedatensatz bezüglich einer ersten Richtung und bezüglich einer zweiten Richtung ausgedehnt ist, weiterhin umfasst das Verfahren das Entfernen einer zweidimensionalen Schicht des dreidimensionalen Mammographiedatensatzes mittels der Recheneinheit, wobei die zweidimensionale Schicht bezüglich der ersten Richtung und der zweiten Richtung ausgedehnt ist. Die erste Richtung ist insbesondere orthogonal zur zweiten Richtung. Der Erfinder hat erkannt, dass die trainierte Generatorfunktion besonders einfach trainiert werden kann, bzw. dass die trainierte Generatorfunktion auch bei wenigen Trainingsdaten besonders genaue Ergebnisse erzeugen kann, wenn nur dreidimensionale Mammographiedatensätze mit einer festen Anzahl von schichten verarbeitet wird. Daher ist es vorteilhaft, aus dreidimensionalen Mammographiedatensätzen zweidimensionale Schichten zu entfernen.

Nach einem weiteren Aspekt der Erfindung wird die zweidimensionale Schicht basierend auf der Position der Brustwarze im dreidimensionalen Mammographiedatensatz bestimmt. Insbesondere wird die dreidimensionale Schicht so bestimmt, dass der Abstand der dreidimensionalen Schicht zu der Position der Brustwarze größer als ein gegebener Schwellenwert ist, wobei der Abstand in Pixeln bzw. in Schichten gemessen wird. Der Erfinder hat erkannt, dass basierend auf der Position der Brustwarze besonders einfach der Mittelpunkt der Brust bestimmt werden kann.

Nach einem weiteren Aspekt der Erfindung wird die zweidimensionale Schicht basierend auf Gewichtungsfaktoren von Voxeln des dreidimensionalen Mammographiedatensatzes bestimmt. Die Gewichtungsfaktoren können insbesondere Angeben, mit welcher Wahrscheinlichkeit ein Voxel eine Läsion abbildet. Insbesondere wird die zweidimensionale Schicht so bestimmt, dass der Abstand der zweidimensionalen Schicht zu einem Voxel mit einem extremalen (insbesondere maximalen) Gewichtungsfaktor größer als ein gegebener Schwellenwert ist, wobei der Abstand in Pixeln bzw. in Schichten gemessen wird. Der Erfinder hat erkannt, dass basierend auf Gewichtungsfaktoren von Voxeln besonders einfach relevante Bereiche des dreidimensionalen Mammographiedatensatzes bestimmt werden können.

Nach einem weiteren Aspekt der Erfindung umfasst der zweidimensionale Mammographiedatensatz ein oder mehrere Pixel, weiterhin umfasst das Verfahren das zweite Bestimmen eines zweidimensionalen Wahrscheinlichkeitsdatensatzes, wobei der zweidimensionale Wahrscheinlichkeitsdatensatz jedem der ein oder mehreren Pixel des zweidimensionalen Mammographiedatensatzes eine Wahrscheinlichkeit zuordnet, dass der jeweilige Pixel eine Läsion abbildet. Der Erfinder hat erkannt, dass basierend auf dem zweidimensionalen Wahrscheinlichkeitsdatensatz eine besonders einfache und schnelle Befundung des zweidimensionalen Mammographiedatensatzes möglich ist.

Die Erfindung betrifft weiterhin ein Bestimmungssystem, umfassend die folgenden Einheiten:

- Schnittstelle, ausgebildet zum Empfangen eines dreidimensionalen Mammographiedatensatzes eines Untersuchungsbereiches,
- Recheneinheit, ausgebildet zum ersten Bestimmen eines zweidimensionalen Mammographiedatensatzes des Untersuchungsbereiches durch Anwendung einer trainierten Generatorfunktion auf den dreidimensionalen Mammographiedatensatz,
   wobei die trainierte Generatorfunktion auf einem trainierten GA-Netzwerk basiert.

Ein solches Bestimmungssystem kann insbesondere dazu ausgebildet sein, die zuvor beschriebenen erfindungsgemäßen Verfahren und ihre Aspekte auszuführen. Das Bestimmungssystem ist dazu ausgebildet, diese Verfahren und ihre Aspekte auszuführen, indem die Schnittstelle und die Recheneinheit ausgebildet sind, die entsprechenden Verfahrensschritte auszuführen.

Die Erfindung betrifft auch ein Computerprogrammprodukt mit einem Computerprogramm sowie ein computerlesbares Medium. Eine weitgehend softwaremäßige Realisierung hat den Vorteil, dass auch schon bisher verwendete Bestimmungssysteme auf einfache Weise durch ein Software-Update nachgerüstet werden können, um auf die erfindungsgemäße Weise zu arbeiten. Ein solches Computerprogrammprodukt kann neben dem Computerprogramm gegebenenfalls zusätzliche Bestandteile wie z. B. eine Dokumentation und/oder zusätzliche Komponenten, sowie Hardware-Komponenten, wie z.B. Hardware-Schlüssel (Dongles etc.) zur Nutzung der Software, umfassen.

Die Erfindung kann auch eine Mammographieeinheit betreffen, wobei die Mammographieeinheit ein zuvor beschriebenes Bestimmungssystem umfasst.

Ein weiterer Aspekt kann auch ein Verfahren zum Bereitstellen einer Generatorfunktion betreffen, insbesondere einer trainierten Generatorfunktion, umfassend die folgenden Verfahrensschritte:

- Erstes Trainings-Empfangen eines dreidimensionalen Trainingsmammographiedatensatzes eines Trainingsuntersuchungsbereiches mittels einer Schnittstelle,
- Erstes Trainings-Bestimmen eines synthetischen zweidimensionalen Trainingsmammographiedatensatzes des Trainingsuntersuchungsbereichs durch Anwendung einer Generatorfunktion auf den dreidimensionalen Trainingsmammographiedatensatz mittels einer Recheneinheit,
- Zweites Trainings-Bestimmen eines ersten Wahrscheinlichkeitswertes durch Anwendung einer Klassifikatorfunktion auf den synthetischen zweidimensionalen Trainingsmammographiedatensatz mittels der Recheneinheit,
- Zweites Trainings-Empfangen eines realen zweidimensionalen Trainingsmammographiedatensatzes mittels der Schnittstelle,
- Drittes Trainings-Bestimmen eines zweiten Wahrscheinlichkeitswertes durch Anwendung der Klassifikatorfunktion auf den realen zweidimensionalen Trainingsmammographiedatensatz mittels der Recheneinheit,
- Anpassen der Parameter der Generatorfunktion basierend auf dem ersten Wahrscheinlichkeitswert und/oder dem zweiten Wahrscheinlichkeitswert mittels der Recheneinheit.
- Bereitstellen der Generatorfunktion mittels der Schnittstelle.

Insbesondere können auch die Parameter der Klassifikatorfunktion basierend auf dem ersten Wahrscheinlichkeitswert und/oder dem zweiten Wahrscheinlichkeitswert angepasst werden. Insbesondere kann das Anpassen der Parameter der Generatorfunktion weiterhin auch auf dem synthetischen zweidimensionalen Trainingsmammographiedatensatz und/oder dem dreidimensionalen Trainingsmammographiedatensatz basieren.

Ein Trainingsmammographiedatensatz kann hierbei alle Merkmale und vorteilhaften Weiterbildungen eines Mammographiedatensatzes aufweisen. Insbesondere kann ein synthetischer und/oder realer zweidimensionaler Trainingsmammographiedatensatz alle Merkmale und vorteilhaften Weiterbildungen eines zweidimensionalen Mammographiedatensatzes aufweisen. Insbesondere kann ein dreidimensionaler Trainingsmammographiedatensatz alle Merkmale und vorteilhaften Weiterbildungen eines dreidimensionalen Mammographiedatensatzes aufweisen. Ein Trainingsuntersuchungsbereich kann hierbei alle Merkmale und vorteilhaften Weiterbildungen eines Untersuchungsbereichs aufweisen.

Ein Mammographiedatensatz bzw. Trainingsmammographiedatensatz wird hierbei insbesondere als synthetisch bezeichnet, wenn er aus einem Mammographiedatensatz bzw. einem Trainingsmammographiedatensatz mit einer abweichenden Dimensionalität bestimmt wird. Ein Mammographiedatensatz bzw. Trainingsmammographiedatensatz wird hierbei insbesondere als real bezeichnet, wenn er auf einer direkten Messung basiert. Ein dreidimensionaler Trainingsmammographiedatensatz ist insbesondere ein realer Trainingsmammographiedatensatz.

Das Verfahren zum Bereitstellen einer Generatorfunktion ist ein Verfahren zum Trainieren einer Generatorfunktion bzw. ein Trainingsverfahren der Generatorfunktion.

Das Bestimmungssystem kann insbesondere dazu ausgebildet sein, das zuvor beschriebenen Verfahren zum Bereitstellen einer Generatorfunktion und seine Aspekte auszuführen. Das Bestimmungssystem ist dazu ausgebildet, dieses Verfahren zum Bereitstellen einer Generatorfunktion und seine Aspekte auszuführen, indem die Schnittstelle und die Recheneinheit ausgebildet sind, die entsprechenden Verfahrensschritte auszuführen.

Ein weiterer Aspekt kann auch ein Computerprogrammprodukt mit einem Computerprogramm betreffen, welches direkt in eine Speichereinheit eines Bestimmungssystems ladbar ist, mit Programmabschnitten, um alle Schritte des Verfahren zum Bereitstellen einer Generatorfunktion und seiner Aspekte auszuführen, wenn die Programmabschnitte von dem Bestimmungssystem ausgeführt werden.

Ein weiterer Aspekt kann auch ein computerlesbares Speichermedium, auf welchem von einem Bestimmungssystem lesbare und ausführbare Programmabschnitte gespeichert sind, um alle Schritte des Verfahren zum Bereitstellen einer Generatorfunktion und seiner Aspekte auszuführen, wenn die Programmabschnitte von dem Bestimmungssystem ausgeführt werden.

Ein weiterer Aspekt kann auch ein Computerprogrammprodukt oder ein computerlesbares Speichermedium umfassend eine trainierte Generatorfunktion umfassen, wobei die Generatorfunktion mittels des Verfahrens zum Bereitstellen einer Generatorfunktion und seiner Aspekte trainiert wurde.

Ein Mammographiedatensatz ist ein Bilddatensatz einer menschlichen Brust, insbesondere einer weiblichen Brust. Ein Mammographiedatensatz ist das Ergebnis einer Röntgenmammographie, einer Magnetresonanz-Mammographie, einer Tomosynthese oder einer Mammosonographie.

Ein Bilddatensatz umfasst zumindest ein Bild, weiterhin kann ein Bilddatensatz noch weitere Daten, insbesondere Metadaten umfassen. Ein Bilddatensatz kann insbesondere mit dem Bild identisch sein. Ein zweidimensionaler Bilddatensatz umfasst zumindest ein zweidimensionales Bild, insbesondere umfasst ein zweidimensionaler Bilddatensatz kein weiteres Bild mit einer abweichenden Dimensionalität. Ein dreidimensionaler Bilddatensatz umfasst zumindest ein dreidimensionales Bild, insbesondere umfasst ein dreidimensionaler Bilddatensatz kein weiteres Bild mit einer abweichenden Dimensionalität. Ein zweidimensionaler Bilddatensatz ist insbesondere mit einem zweidimensionalen Bild identisch. Ein dreidimensionaler Bilddatensatz ist insbesondere mit einem dreidimensionalen Bild identisch. Metadaten sind hierbei Informationen über die bildgebende Untersuchung, die Grundlage für den Bilddatensatz ist, die keine Bilder oder Bilddaten sind, beispielsweise Patientendaten oder Daten über das verwendete Protokoll. Ein Datensatz bzw. ein Bilddatensatz ist insbesondere in eine Richtung ausgedehnt, wenn die in Pixeln oder Voxel gemessene Ausdehnung des Bilddatensatzes in diese Richtung größer als eins ist. Ein dreidimensionaler Bilddatensatz, dessen in Voxeln gemessene Ausdehnung in eine Richtung genau eins ist, kann auch als zweidimensionaler Bilddatensatz aufgefasst werden.

Ein Bilddatensatz umfasst eine Mehrzahl von Pixeln oder Voxeln. Hierbei werden die Begriffe Pixel und Voxel synonym verwendet, lassen also insbesondere keinen Rückschluss auf eine Dimensionalität zu. Jedem der Pixel oder der Voxel ist ein Intensitätswert zugeordnet, der vorzugsweise einem Röntgenabsorptionswert entspricht.

Ein GA-Algorithmus ("GA" ist ein Akronym für "generative adversarial", eine deutsche Übersetzung ist "erzeugend und gegnerisch") umfasst eine Generatorfunktion und eine Klassifikatorfunktion. Hierbei erzeugt die Generatorfunktion synthetische Daten, und die Klassifikatorfunktion unterscheidet zwischen synthetischen und realen Daten. Insbesondere wird durch ein Training der Generatorfunktion und/oder der Klassifikatorfunktion erreicht, dass auf der einen Seite die Generatorfunktion synthetische Daten erzeugt, die durch die Klassifikatorfunktion fälschlicherweise als real eingestuft werden, auf der anderen Seite die Klassifikatorfunktion möglichst gut zwischen realen Daten und synthetischen Daten unterscheiden kann. Spieltheoretisch kann ein GA-Algorithmus auch als Nullsummenspiel aufgefasst werden. Das Training der Generatorfunktion und/oder der Klassifikatorfunktion basiert insbesondere auf der Minimierung jeweils einer Kostenfunktion.

Falls die Generatorfunktion und die Klassifikatorfunktion durch ein Netzwerk gegeben sind, insbesondere durch ein künstliches neuronales Netzwerk, dann wird der GA-Algorithmus auch als GA-Netzwerke (auch "GAN", englisches Akronym für "generative adversarial networks", eine deutsche Übersetzung ist "erzeugende gegnerische Netzwerke") bezeichnet. Diese sind insbesondere aus der Druckschrift Ian J. Goodfellow, "Generative Adversarial Networks", arxiv 1406.2661 (2014) bekannt. Die Minimierung der Kostenfunktion kann insbesondere mittels Rückpropagation erfolgen.

Die trainierte Generatorfunktion basiert insbesondere derart auf einem GA-Algorithmus oder auf GA-Netzwerken, dass die trainierte Generatorfunktion mit der Generatorfunktion des GA-Algorithmus oder der GA-Netzwerke identisch ist.

Eine Funktion umfasst einen Operator oder eine Unterfunktion, wenn diese Funktion eine Verkettung des Operators oder der Unterfunktion mit einer oder mehreren weiteren Funktionen ist. Insbesondere umfasst die erste Teilfunktion einen dreidimensionalen Faltungsoperator, wenn die erste Teilfunktion eine Verkettung des dreidimensionalen Faltungsoperators mit einer oder mehreren weiteren Funktionen ist. Insbesondere umfasst die zweite Teilfunktion einen zweidimensionalen Faltungsoperator, wenn die erste Teilfunktion eine Verkettung des zweidimensionalen Faltungsoperators mit einer oder mehreren weiteren Funktionen ist.

Ein d-dimensionaler Faltungsoperator (ein englischer Fachbegriff ist "convolutional operator") bildet ein d-dimensionales Eingabearray auf ein oder mehrere d-dimensionale Ausgabearrays ab, indem eine mathematische Faltung mit einem oder mehreren Kernen auf das d-dimensionale Eingabearray angewendet wird. Der eine oder die mehreren Kerne sind hierbei auch d-dimensionale Arrays. Insbesondere sind hierbei die Ausdehnung des Eingabearrays und des Ausgabearrays bezüglich jeder Dimension identisch. Ein d-dimensionales Array ist insbesondere eine d-dimensionale Matrix oder ein d-dimensionaler Bilddatensatz.

Ein d-dimensionaler Bündelungsoperator (ein englischer Fachbegriff ist "pooling operator") bildet ein d-dimensionales Eingabearray auf ein d-dimensionale Ausgabearray ab, wobei die Ausdehnung des Ausgabearrays bezüglich mindestens einer Dimension kleiner ist als die Ausdehnung des Eingabearrays bezüglich dieser Dimension.

Ein d-dimensionaler Entbündelungsoperator (ein englischer Fachbegriff ist "unpooling operator") bildet ein d-dimensionales Eingabearray auf ein d-dimensionale Ausgabearray ab, wobei die Ausdehnung des Ausgabearrays bezüglich mindestens einer Dimension größer ist als die Ausdehnung des Eingabearrays bezüglich dieser Dimension.

Sowohl ein d-dimensionaler Faltungsoperator als auch ein d-dimensionaler Bündelungsoperator und ein d-dimensionaler Entbündelungsoperator können in einem künstlichen neuronalen Netzwerk realisiert sein, insbesondere durch zwei benachbarte Knotenschichten, wobei die erste Knotenschicht das d-dimensionale Eingabearray und die zweite Knotenschicht das eine oder die mehreren d-dimensionalen Ausgabearrays repräsentiert. Der jeweilige Operator ist dann durch die Topologie (Kanten zwischen Knoten der ersten und der zweiten Knotenschicht) und deren Gewichten gegeben. In diesem Fall wird ein d-dimensionale Faltungsoperator auch als d-dimensionale Faltungsschicht (ein englischer Fachbegriff ist "convolutional layer"), ein d-dimensionaler Bündelungsoperator als d-dimensionale Bündelungsschicht (ein englischer Fachbegriff ist "pooling layer") und ein d-dimensionaler Entbündelungsoperator als d-dimensionale Entbündelungsschicht (ein englischer Fachbegriff ist "unpooling layer") bezeichnet. Eine Schicht umfasst also insbesondere zwei benachbarte Knotenschichten und alle Kanten zwischen Knoten der zwei benachbarten Knotenschichten. Zwei unterschiedliche Schichten können eine gemeinsame Knotenschicht aufweisen, in diesem Fall dient der Ausgabewert der ersten Schicht der zweiten Schicht als Eingabewert.

Ein Merkmalsvektor ist eine Menge von Zahlen, welche als Vektor aufgefasst werden kann. Ein englischer Fachbegriff für Merkmalsvektor ist "feature vector". Ein Merkmalsvektor kann auch ein Merkmalskanals sein, ein englischer Fachbegriff für Merkmalskanals ist "feature channel". Insbesondere kann ein Merkmalsvektor auch ein Bilddatensatz, insbesondere ein zweidimensionaler oder dreidimensionaler Bilddatensatz sein. Insbesondere kann der erste Merkmalsvektor auch ein dreidimensionaler Bilddatensatz sein, und der zweite Merkmalsvektor ein zweidimensionaler Bilddatensatz.

Die Verfahren zum Bestimmen eines zweidimensionalen Mammographiedatensatzes und zum Trainieren einer Generatorfunktion sind insbesondere computerimplementierte Verfahren.

Im Folgenden wird die Erfindung anhand der in den Figuren dargestellten Ausführungsbeispiele sowie anhand von Pseudocode näher beschrieben und erläutert.

Fig. 1    zeigt ein Flussdiagramm des Verfahrens zum Bestimmen eines zweidimensionalen Mammographiedatensatzes,

Fig. 2    zeigt ein Bestimmungssystem,

Fig. 3    zeigt ein Flussdiagramm eines Verfahrens zum Trainieren einer Generatorfunktion,

Fig. 4    zeigt den Datenfluss eines Verfahrens zum Trainieren einer Generatorfunktion,

Fig. 5    zeigt ein erstes Ausführungsbeispiel einer Generatorfunktion,

Fig. 6    zeigt ein zweites Ausführungsbeispiel einer Generatorfunktion,

Fig. 7    zeigt eine zweidimensionale Faltungsschicht

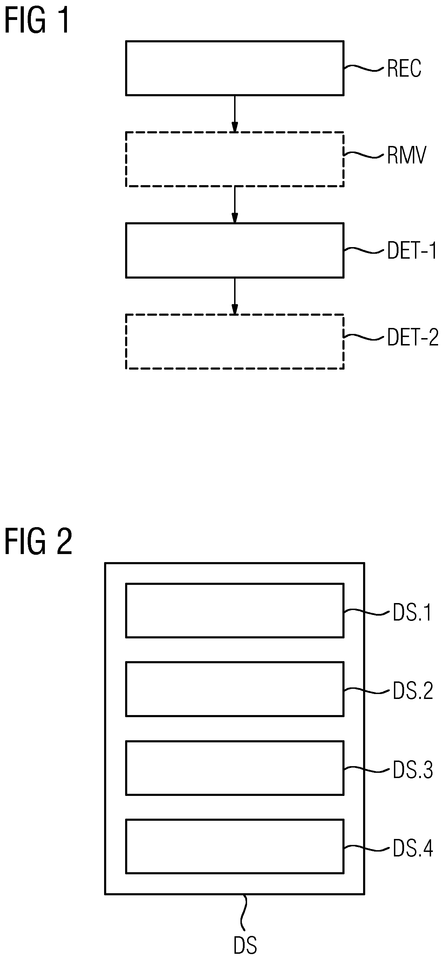

Fig. 1 zeigt ein Flussdiagramm eines Ausführungsbeispiels des Verfahrens zum Bestimmen eines zweidimensionalen Mammographiedatensatzes $G\_x$, $G\_x'$. Optionale Schritte des Verfahrens sind hierbei durch gestrichelte Linien dargestellt.

Weiterhin zeigt Tabelle A zugehörigen Pseudocode des Ausführungsbeispiels.

| Tabelle A: Pseudocode zum Bestimmen eines zweidimensionalen Mammographiedatensatzes $G\_x$, $G\_x'$ | |
| --- | --- |
| A.1 | func mammo_2d_from_3d(image_3d x, int direction): |
| A.2 | int mid_layer = nipple_layer(x) |
| A.3 | int first_layer = mid_layer - N/2 |
| A.4 | int last_layer = mid_layer + 1 - N/2 |
| A.5 | image_3d x' = select_layers(x, direction, first_layer, last_layer) |
| A.6 | image_2d G_ x = G.convert(x') |
| A.7 | image_2d GPM_x = G.probability(x') |
| A.8 | return G_x, GPM_x |

Der erste Schritt des dargestellten Ausführungsbeispiels ist das Empfangen REC eines dreidimensionalen Mammographiedatensatzes x eines Untersuchungsbereiches mittels einer Schnittstelle DS.1. Dieser Schritt entspricht der Codezeile A.1. Bei dem dreidimensionalen Mammographiedatensatz x handelt es sich hierbei um eine digitale Brusttomosynthese. Der dreidimensionale Mammographiedatensatz x umfasst in einem dreidimensionalen Gitter angeordnete Voxel, wobei die Ausdehnung des dreidimensionalen Mammographiedatensatz x in eine erste Richtung $L_1$ Voxel entspricht, die Ausdehnung in eine zweite Richtung $L_2$ Voxel entspricht, wobei die zweite Richtung orthogonal zur ersten Richtung ist, und die Ausdehnung in eine dritte Richtung $L_3$ Voxel entspricht, wobei die dritte Richtung orthogonal zur ersten Richtung und orthogonal zur dritten Richtung ist. Insgesamt umfasst der dreidimensionale Mammographiedatensatz x also $L_1 \cdot L_2 \cdot L_3$ Voxel.

Im dargestellten Ausführungsbeispiel umfasst der dreidimensionale Mammographiedatensatz x noch eine Information, welche Richtung der ersten Richtung, der zweiten Richtung und der dritten Richtung im zweidimensionalen Mammographiedatensatz G_x, G_x' nicht mehr vorhanden sein soll. In der weiteren Beschreibung wird davon ausgegangen, dass dies in diesem Fall die dritte Richtung ist, so dass der zweidimensionale Mammographiedatensatz G_x, G_x' genau $L_1 \cdot L_2$ Pixel umfasst.

Der zweite Schritt des dargestellten Ausführungsbeispiels ist das Entfernen RMV einer zweidimensionalen Schicht w.1, w.2, w.7, w.8 des dreidimensionalen Mammographiedatensatzes x mittels der Recheneinheit DS.2, wobei die zweidimensionale Schicht w.1, w.2, w.7, w.8 bezüglich der ersten Richtung u und der zweiten Richtung v ausgedehnt ist. Der Schritt des Entfernens RMV ist ein optionaler Schritt, er entspricht den Codezeilen A.3 bis A.5 im Pseudocode.

In diesem Ausführungsbeispiel wird die zweidimensionale Schicht w.1, w.2, w.7, w.8 basierend auf der Position der Brustwarze im dreidimensionalen Mammographiedatensatz x bestimmt (vgl. Codezeile A.2). Alternativ kann die zweidimensionale Schicht w.1, w.2, w.7, w.8 basierend auf Gewichtungsfaktoren von Voxeln des dreidimensionalen Mammographiedatensatzes x bestimmt werden.

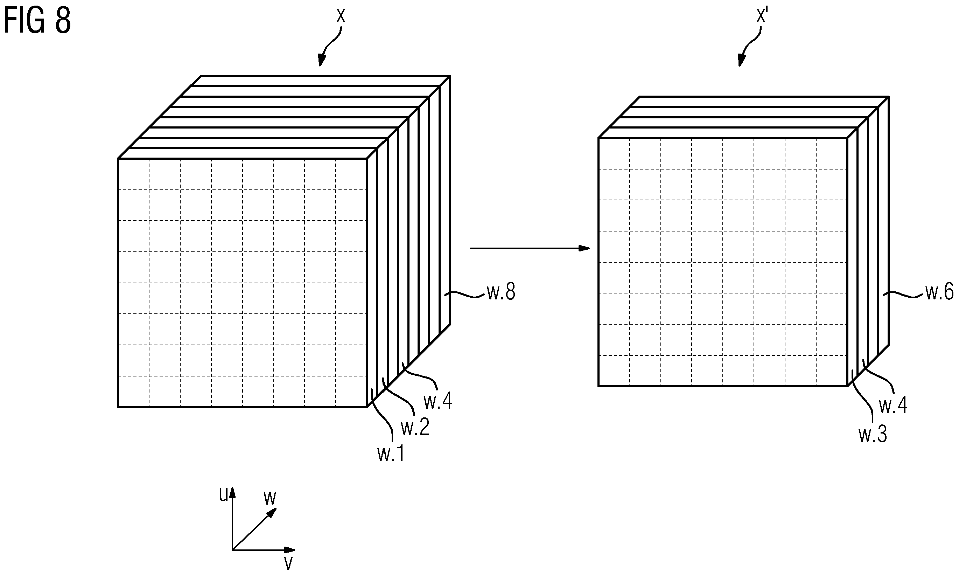

Der Schritt des Entfernens RMV einer zweidimensionalen Schicht w.1, w.2, w.7, w.8 wird in der Beschreibung zur Fig. 8 genauer erläutert. Das Resultat der einmaligen oder mehrmaligen Durchführung des Entfernens ist ebenfalls ein dreidimensionaler Mammographiedatensatz x'.

Die im Folgenden beschriebenen Schritte des Ausführungsbeispiels sind davon unabhängig, ob der optionale Schritt des Entfernens RMV durchgeführt wurde. In anderen Worten können sie also sowohl auf einen dreidimensionalen Mammographiedatensatz x angewendet werden, von dem keine zweidimensionale Schicht w.1, ..., w.8 entfernt wurde, als auch auf einen dreidimensionalen Mammographiedatensatz x', von dem mindestens eine zweidimensionale Schicht w.1, w.2, w.7, w.8 entfernt wurde. Hierbei ist es aber vorteilhaft, eine in der Generatorfunktion G vorhandene Projektionsfunktion G.PL auf die Anzahl der verwendeten zweidimensionalen Schichten w.1, ..., w.8 anzupassen.

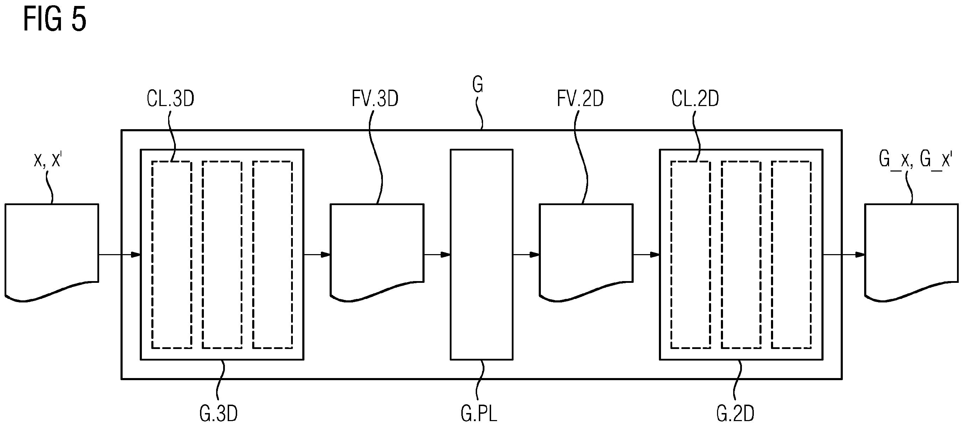

Der dritte Schritt des dargestellten Ausführungsbeispiels ist das erste Bestimmen DET-1 eines zweidimensionalen Mammographiedatensatzes G_x, G_x' des Untersuchungsbereiches durch Anwendung einer trainierten Generatorfunktion G auf den dreidimensionalen Mammographiedatensatz x, x' mittels einer Recheneinheit DS.2, wobei die trainierte Generatorfunktion G auf einem trainierten GA-Algorithmus basiert. Der zweidimensionale Mammographiedatensatz G_x, G_x' ist hier insbesondere ein synthetischer zweidimensionaler Mammographiedatensatz G_x, G_x'. Der Schritt des ersten Bestimmens DET-1 entspricht der Codezeile A.6. Details sowie alternative Ausführungsbeispiele zum Schritt des ersten Bestimmens DET-1 werden in der Beschreibung zur Fig. 5 erläutert.

Bei der Generatorfunktion G handelt es sich in diesem Ausführungsbeispiel um ein künstliches neuronales Netzwerk, alternativ kann die Generatorfunktion G aber auch auf einem Perzeptron, einer Stützvektormaschine (ein englischer Fachbegriff ist "support vector machine", kurz "SVM"), ein Zufallswald (ein englischer Fachbegriff ist "random forest") oder einer Regressionsfunktion basieren.

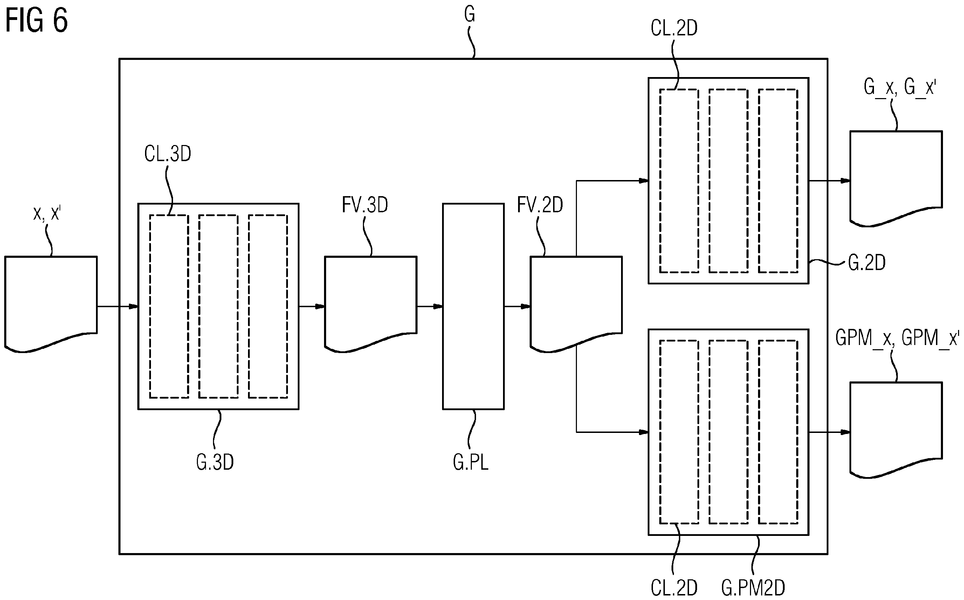

Der vierte Schritt des dargestellten Ausführungsbeispiels ist das zweites Bestimmen DET-2 eines zweidimensionalen Wahrscheinlichkeitsdatensatzes GPM_x, GPM_x', wobei der zweidimensionale Wahrscheinlichkeitsdatensatz GPM_x, GPM_x' jedem der ein oder mehreren Pixel des zweidimensionalen Mammographiedatensatzes G_x, G_x' eine Wahrscheinlichkeit zuordnet, dass der jeweilige Pixel eine Läsion abbildet. Das zweite Bestimmen DET-2 ist hierbei ein optionaler Schritt. Der Schritt des ersten Bestimmens DET-1 entspricht der Codezeile A.7. Details sowie alternative Ausführungsbeispiele zum Schritt des zweiten Bestimmens DET-2 werden in der Beschreibung zur Fig. 6 erläutert.

Fig. 2 zeigt ein Bestimmungssystem DS zum Bestimmen eines zweidimensionalen Mammographiedatensatzes G_x, G_x'. Das hier dargestellte Bestimmungssystem DS ist dazu ausgelegt, ein erfindungsgemäßes Verfahren auszuführen. Dieses Bestimmungssystem umfasst eine Schnittstelle DS.1, eine Recheneinheit DS.2, eine Speichereinheit DS.3 sowie eine Ein- und Ausgabeeinheit DS.4.

Bei dem Bestimmungssystem DS kann es sich insbesondere um einen Computer, einen Mikrocontroller oder um einen integrierten Schaltkreis handeln. Alternativ kann es sich bei dem Bestimmungssystem um einen realen oder virtuellen Verbund von Computern handeln (ein englischer Fachbegriff für einen realen Verbund ist "Cluster", ein englischer Fachbegriff für einen virtuellen Verbund ist "Cloud"). Insbesondere kann das Bestimmungssystem DS auch Teil einer bildgebenden Mammographieeinheit sein, alternativ kann das Bestimmungssystem DS auch selbst eine bildgebende Mammographieeinheit sein.

Bei einer Schnittstelle DS.1 kann es sich um eine Hardware- oder Softwareschnittstelle handeln (beispielsweise PCI-Bus, USB oder Firewire). Eine Recheneinheit DS.2 kann Hardware-Elemente oder Software-Elemente aufweisen, beispielsweise einen Mikroprozessor oder ein sogenanntes FPGA (englisches Akronym für "Field Programmable Gate Array"). Eine Speichereinheit DS.3 kann als nicht dauerhafte Arbeitsspeicher (Random Access Memory, kurz RAM) oder als dauerhafter Massenspeicher (Festplatte, USB-Stick, SD-Karte, Solid State Disk) realisiert sein. Eine Ein- und Ausgabeeinheit DS.4 umfasst wenigstens eine Eingabeeinheit und/oder wenigstens eine Ausgabeeinheit.

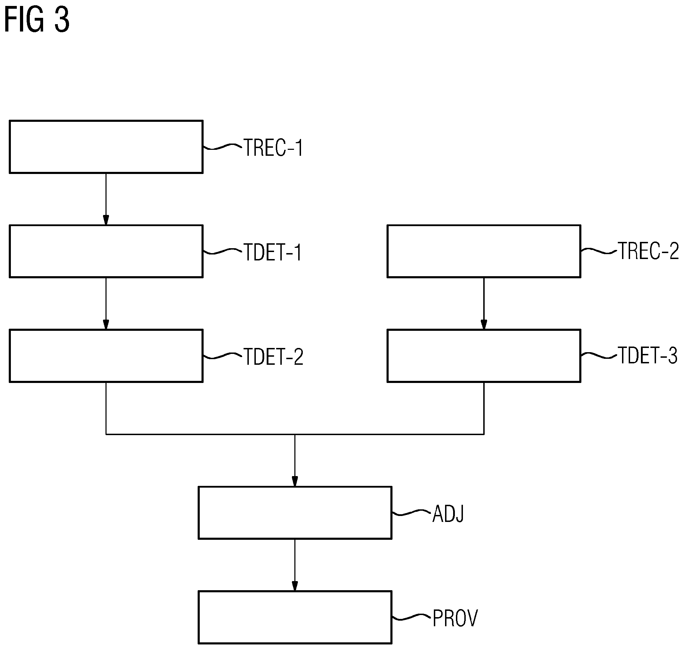

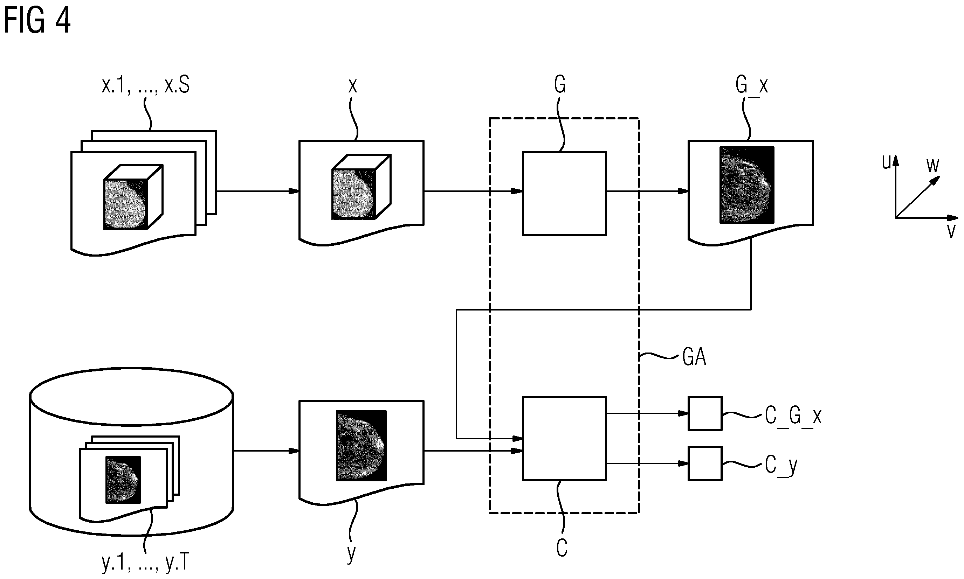

Fig. 3 zeigt ein Flussdiagramm eines Verfahrens zum Bereitstellen einer Generatorfunktion, Fig. 4 zeigt den Datenfluss beim Verfahren zum Bereitstellen einer Generatorfunktion G. Die Generatorfunktion G wird hierbei anhand von einer Menge von dreidimensionalen Trainingsmammographiedatensätzen x.1, ..., x.S und einer Menge von realen zweidimensionalen Trainingsmammographiedatensätzen y.1, ..., y.T trainiert.

Das Training der Generatorfunktion erfolgt in diesem Ausführungsbeispiel schrittweise, es wird also in jedem Trainingsschritt nur genau ein dreidimensionaler Trainingsmammographiedatensatz x der dreidimensionalen Trainingsmammographiedatensätze x.1, ..., x.S und genau ein realer zweidimensionaler Trainingsmammographiedatensatz y der realen zweidimensionalen Trainingsmammographiedatensätze y.1, ..., y.T gleichzeitig verwendet, diese Trainingsschritt kann aber beliebig oft wiederholt werden, um eine besser trainierte Generatorfunktion G zu erhalten. Alternativ ist es auch möglich, in einem Trainingsschritt mehrere der der dreidimensionalen Trainingsmammographiedatensätze x.1, ..., x.S und/oder mehrere der realen zweidimensionalen Trainingsmammographiedatensätze y.1, ..., y.T zu verwenden.

Der erste Schritt des dargestellten Verfahrens ist das erste Trainings-Empfangen TREC-1 eines dreidimensionalen Trainingsmammographiedatensatzes x eines Trainingsuntersuchungsbereiches mittels einer Schnittstelle DS.1. Der dreidimensionale Trainingsmammographiedatensatz x ist hierbei einer der dreidimensionalen Trainingsmammographiedatensätze x.1, ..., x.S.

Der zweite Schritt des dargestellten Verfahrens ist das erste Trainings-Bestimmen TDET-1 eines synthetischen zweidimensionalen Mammographiedatensatzes G_x des Trainingsuntersuchungsbereichs durch Anwendung der Generatorfunktion G auf den dreidimensionalen Trainingsmammographiedatensatz x mittels einer Recheneinheit DS.2. Hierbei ist der dreidimensionale Trainingsmammographiedatensatz x der Eingabewert der Generatorfunktion G, der synthetische zweidimensionale Mammographiedatensatz G_x ist der durch die Generatorfunktion G erzeugte Ausgabewert G(x).

Zum Training der Generatorfunktion G wird weiterhin eine Klassifikatorfunktion C benötigt. Die Klassifikatorfunktion C bildet einen realen zweidimensionalen Trainingsmammographiedatensatz y oder einen synthetischen zweidimensionalen Mammographiedatensatz G_x auf eine reelle Zahl zwischen 0 und 1 abbildet, insbesondere auf einen Wahrscheinlichkeitswert. Dieser Wahrscheinlichkeitswert entspricht der Wahrscheinlichkeit, dass der Eingabedatensatz der Klassifikatorfunktion C ein Element der Menge von zweidimensionalen Trainingsmammographiedatensätzen y.1, ..., y.T ist. Die Generatorfunktion G und die Klassifikatorfunktion C bilden zusammen mit geeigneten Kostenfunktionen ein GA-Netzwerk.

Der dritte Schritt des dargestellten Ausführungsbeispiels ist das zweite Trainings-Bestimmen TDET-2 eines ersten Wahrscheinlichkeitswertes C_G_x durch Anwendung der Klassifikatorfunktion C auf den synthetischen zweidimensionalen Mammographiedatensatz G_x mittels der Recheneinheit DS.2. Hierbei erhält die Klassifikatorfunktion C als Eingabewert den synthetischen zweidimensionalen Mammographiedatensatz G_x und erzeugt als Ausgabewert den ersten Wahrscheinlichkeitswert C_G_x.

Der vierte Schritt des dargestellten Ausführungsbeispiels ist das zweite Trainings-Empfangen TDET-2 eines realen zweidimensionalen Trainingsmammographiedatensatzes y mittels der Schnittstelle DS.1. Der reale zweidimensionale Trainingsmammographiedatensatz y ist hierbei einer der realen zweidimensionalen Trainingsmammographiedatensätze y.1, ..., y.T.

Der vierte Schritt des dargestellten Ausführungsbeispiels ist das dritte Trainings-Bestimmen TDET-3 eines zweiten Wahrscheinlichkeitswertes C_y durch Anwendung der Klassifikatorfunktion C auf den realen zweidimensionalen Trainingsmammographiedatensatz y mittels der Recheneinheit DS.2. Hierbei erhält die Klassifikatorfunktion C als Eingabewert einen realen zweidimensionalen Mammographiedatensatz y der zweidimensionalen Trainingsmammographiedatensätze y.1, ..., y.T, und erzeugt als Ausgabewert den zweiten Wahrscheinlichkeitswert C_y.

Die Schritte des ersten Trainings-Empfangens TREC-1, des ersten Trainings-Bestimmens TDET-1 und des zweiten Trainings-Bestimmens TDET-2 werden hierbei in der aufgeführten Reihenfolge durchgeführt, sie bilden eine erste Gruppe. Weiterhin werden die Schritte des zweiten Trainings-Empfangens TREC-2 und des dritten Trainings-Bestimmens TDET-3 in der aufgeführten Reihenfolge durchgeführt, sie bilden eine zweite Gruppe. Diese beiden Gruppen von Schritten sind aber unabhängig voneinander, d.h. die Schritte des zweiten Trainings-Empfangens TREC-2 und des dritten Trainings-Bestimmens TDET-3 können auch jeweils vor einem der Schritte des ersten Trainings-Empfangens TREC-1, des ersten Trainings-Bestimmens TDET-1 und des zweiten Trainings-Bestimmens TDET-2 durchgeführt werden. Insbesondere können die Schritte des ersten Trainings-Empfangens TREC-1 und des zweiten Trainings-Empfangens TREC-2 jeweils vor den Schritten des ersten Trainings-Bestimmens TDET-1, des zweiten Trainings-Bestimmens TDET-2 und des dritten Trainings-Bestimmens TDET-3 durchgeführt werden.

Der sechste Schritt des dargestellten Ausführungsbeispiels ist das Anpassen ADJ der Parameter der Generatorfunktion G basierend auf dem ersten Wahrscheinlichkeitswert C_G_x und auf dem zweiten Wahrscheinlichkeitswert C_y. Insbesondere werden hierbei auch Parameter der Klassifikatorfunktion C angepasst. In diesem Ausführungsbeispiel werden hierfür zwei Kostenfunktionen, die von C_G_x und C_y abhängen, durch Anpassung von Parametern der Generatorfunktion G und der Klassifikatorfunktion C minimiert. Durch eine geeignete Wahl der Kostenfunktion wird die Generatorfunktion G so trainiert, dass die Klassifikatorfunktion C nicht entscheiden kann, ob G_x aus der Menge der realen zweidimensionalen Trainingsmammographiedatensätze y.1, ..., y.T gewählt ist, also C_G_x = 0.5 ist. Hieraus ergibt sich auch der englische Fachbegriff "generative-adversarial" des Trainingsverfahrens, da die Generatorfunktion G synthetische Daten erzeugt ("generativ"), und die Generatorfunktion G und die Klassifikatorfunktion C gegeneinander trainiert werden ("adversarial").

Im Folgenden werden jeweils mögliche Kostenfunktionen der Generatorfunktion G und der Klassifikatorfunktion C beschrieben. Es ist aber natürlich jederzeit möglich, andere Kostenfunktionen zu verwenden.

Die Kostenfunktion $K_C$ der Klassifikatorfunktion C ist in diesem Ausführungsbeispiel gegeben durch

$$K_C \propto BCE(C\_y, 1) + BCE(C\_G\_x, 0)$$

wobei BCE die binäre Kreuzentropie (ein englischer Fachbegriff ist "binary cross-entropy") bezeichnet:

$$BCE(z, z') = z' \cdot \log(z) + (1 - z') \cdot \log(1 - z)$$

Insbesondere ist also die Kostenfunktion $K_C$ der Klassifikatorfunktion C gegeben durch:

$$K_C(C\_y, C\_G\_x) \propto \log(C\_y) + \log(1 - C\_G\_x)$$

Es ist ebenfalls möglich, einen Trainingsschritt nicht nur mit einem dreidimensionalen Trainingsmammographiedatensatz x und einem realen zweidimensionalen Trainingsmammographiedatensatz y durchzuführen, sondern auch mit mehreren dreidimensionalen Trainingsmammographiedatensätzen und/oder mehreren realen zweidimensionalen Trainingsmammographiedatensätzen. In diesem Fall ist die Kostenfunktion gegeben durch

$$K_C \propto \frac{1}{J} \sum_{j=1}^{J} \log(C\_y_j) + \frac{1}{I} \sum_{i=1}^{I} \log(1 - C\_G\_x_i)$$

wobei I die Anzahl der verwendeten dreidimensionalen Trainingsmammographiedatensätze bezeichnet (mit $I \leq S$), wobei J die Anzahl der verwendeten realen zweidimensionalen Trainingsmammographiedatensätze bezeichnet (mit $J \leq T$), wobei $C\_y_j$ die Anwendung der Klassifikatorfunktion C auf den j-ten realen zweidimensionalen Trainingsmammographiedatensatz bezeichnet, und wobei $C\_G\_x_i$ die Anwendung der Klassifikatorfunktion C auf das Ergebnis der Anwendung der Generatorfunktion auf den i-ten dreidimensionalen Trainingsmammographiedatensatz bezeichnet.

Die Kostenfunktion $K_G$ für die Generatorfunktion G ist gegeben durch

$$K_G = \alpha \cdot K_{proj} + \beta \cdot K_{cont} + \gamma \cdot K_{adver},$$

wobei der Anteil $K_{proj}$ eine Abweichung des synthetischen zweidimensionalen Mammographiedatensatzes G_x von einer Projektion des dreidimensionalen Trainingsmammographiedatensatzes x quantifiziert, wobei $K_{cont}$ einen großen Kontrastunterschied zwischen einem Bereich mit einer Läsion und der Umgebung bevorzugt, und wobei $K_{adver}$ derart gewählt ist, dass die Klassifikatorfunktion die synthetischen zweidimensionalen Trainingsmammographiedatensätze y.1, ..., y.T als real klassifiziert. Die Parameter $\alpha$, $\beta$ und $\gamma$ sind reelle Zahlen, die zur Gewichtung der einzelnen Beiträge zur Kostenfunktion frei gewählt werden können. Im vorliegenden Ausführungsbeispiel werden die Parameter als $\alpha = \beta = \gamma = 1$ gewählt. Der Anteil $K_{cont}$ ist hierbei optional (es kann also $\beta = 0$ gewählt werden).

Die Abweichung des synthetischen zweidimensionalen Mammographiedatensatz G_x von einer Projektion des dreidimensionalen Trainingsmammographiedatensatzes x lässt sich beispielsweise durch die folgende Anteil $K_{proj}$ quantifizieren:

$$K_{proj}(x) = [Pr(x) - G(x)]^2$$

Werden bei einem Trainingsschritt mehrere Trainingsdatensätze verwendet, so wird der Anteil $K_{proj}$ der Kostenfunktion $K_G$ über die mehreren Trainingsdatensätze gemittelt:

$$K_{proj}(x_1, \ldots, x_N) = \frac{1}{N} \cdot \left( \sum_{n=1}^{N} [Pr(x_n) - G(x_n)]^2 \right)$$

Hierbei bezeichnet Pr(x) die Anwendung eines Projektionsoperators auf den dreidimensionalen Trainingsmammographiedatensatz x, insbesondere eines Projektionsoperators bezüglich der dritten Richtung w, wobei der Projektionsoperator einen dreidimensionalen Datensatz auf einen zweidimensionalen Datensatz abbildet. In diesem Ausführungsbeispiel ist der Projektionsoperator eine Maximalintensitätsprojektion (englisch "maximal intensity projection", kurz "MIP") bezüglich der dritten Richtung x als Projektionsrichtung. Alternativ kann es sich auch um eine Projektion auf einen durchschnittlichen Wert entlang der Projektionsrichtung handeln, oder um eine multiplanare Rekonstruktion (englisch "multi planar reformat", kurz "MPR").

Durch diesen Beitrag in der Kostenfunktion $K_G$ wird erreicht, dass der dreidimensionale Trainingsmammographiedatensatz x auf einen synthetischen zweidimensionalen Mammographiedatensatz G_x abgebildet wird, der auch tatsächlich einer zweidimensionalen Projektion Pr(x) des dreidimensionalen Trainingsmammographiedatensatzes x entspricht, und nicht nur einem realen zweidimensionalen Trainingsmammographiedatensatz y.1, ..., y.T ähnlich ist.

Durch die folgende Beitrag $K_{cont}$ zur Kostenfunktion $K_G$ kann beispielsweise ein hoher Kontrast zwischen Bildbereichen, die eine Läsion umfassen, und den restlichen Bildbereichen erreicht werden:

$$K_{cont}(x, m) = -SNR(Pr(m), G(x))$$

Werden bei einem Trainingsschritt mehrere Trainingsdatensätze verwendet, so wird der Anteil $K_{proj}$ der Kostenfunktion $K_G$ über die mehreren Trainingsdatensätze gemittelt:

$$K_{cont}(x_1, \ldots, x_N, m_1, \ldots, m_N) = -\frac{1}{N} \cdot \left( \sum_{n=1}^{N} SNR(Pr(m_n), G(x_n)) \right)$$

Hierbei bezeichnet m bzw. $m_n$ eine Trainingssegmentierung des dreidimensionalen Trainingsmammographiedatensatzes x bzw. $x_n$, bei der eine oder mehrere Läsionen im dreidimensionalen Trainingsmammographiedatensatz x bzw. $x_n$ segmentiert wurden. Die Trainingssegmentierung umfasst hierbei Voxel, denen der Wert 1 zugeordnet ist, wenn das jeweilige Voxel eine Läsion abbildet, und denen der Wert 0 zugeordnet ist, wenn das jeweilige Voxel keine Läsion abbildet. Die Projektionsfunktion kann hierbei auch wieder eine Maximalintensitätsprojektion sein, die Projektionsfunktion kann weiterhin auch von dem dreidimensionalen Trainingsmammographiedatensatz x abhängen. Beispielsweise kann die Projektionsfunktion eine Maximalintensitätsprojektion derart berechnen, dass die relevanten Voxel mit der maximalen Intensität basierend auf dem dreidimensionalen Trainingsmammographiedatensatz x bestimmt werden, aber dann die Pixelwerte der Trainingssegmentierung m projiziert werden. Insbesondere ist Pr(m) also eine zweidimensionale Segmentierung.

Die Funktion SNR berechnet hierbei ein Signal-RauschVerhältnis (englisch "signal-to-noise ratio"), in diesem Ausführungsbeispiel ist die Funktion gegeben durch

$$SNR(Pr(m), G(x)) = \frac{L_1 L_2 - \sum Pr(m)}{\sum Pr(m)} \cdot \frac{Pr(m) \cdot G(x)}{[1 - Pr(m)] \cdot G(x)}$$

wobei das Produkt von zweidimensionalen Datensätzen bzw. Bildern pixelweise ausgeführt wird. Hierbei bezeichnet $L_1$ die Ausdehnung der Datensätze in die erste Richtung u, und $L_2$ die Ausdehnung der Datensätze in die zweite Richtung v, und $\sum Pr(m)$ bezeichnet die Zahl der Pixel in Pr(m), denen als Intensität eine 1 zugeordnet ist. Die Funktion SNR berechnet hier also das Verhältnis des Durchschnitts der Intensitätswerte von G x im segmentierten Bereich zum Durchschnitt der Intensitätswerte von G_x außerhalb des segmentierten Bereichs. Alternativ kann die Funktion SNR auch auf der Standardabweichung der Intensitätswerte von G_x außerhalb des segmentierten Bereichs basieren.

Die Minimierung des Beitrag $K_{cont}$ zur Kostenfunktion $K_G$ führt also dazu, dass Läsionen im synthetischen zweidimensionalen Mammographiedatensatz G_x durch hohe Intensitätswerte gekennzeichnet werden, und daher besonders einfach diagnostiziert werden können.

Der Beitrag $K_{adver}$ zur Kostenfunktion $K_G$ führt dazu, dass die generierten synthetischen zweidimensionalen Mammographiedatensätze G_x möglichst ähnlich den realen zweidimensionalen Trainingsmammographiedatensätzen y.1, ..., y.T sind, indem die synthetischen zweidimensionalen Mammographiedatensätze G_x derart generiert werden, dass die Klassifikatorfunktion diese nicht von den realen zweidimensionalen Trainingsmammographiedatensätzen y.1, ..., y.T unterscheiden kann. Für diesen Beitrag kann wieder die binäre Kreuzentropie gewählt werden

$$K_{adver}(C\_G\_x) = BCE(C\_G\_x,1) = -\log(C\_G\_x) = -\log(C(G(x)))$$

wobei auch hier wieder über mehrere Trainingsdatensätze gemittelt werden kann:

$$K_{adver}(G\_C\_x_1, \ldots, G\_C\_x_N) = -\frac{1}{N} \cdot \left( \sum_{n=1}^{N} \log(G\_C\_x_n) \right)$$

Die Optimierung erfolgt hier also so, dass ein hoher erster Wahrscheinlichkeitswert G_C_x nahe 1 die Kostenfunktion minimiert.

Wenn die Generatorfunktion G basierend auf einem dreidimensionalen Trainingsmammographiedatensatz x neben einem synthetischen zweidimensionalen Mammographiedatensatz G_x noch einen Wahrscheinlichkeitsdatensatz GPM_x erzeugt, kann der Kostenfunktion $K_G$ noch additiv vom Beitrag $K_{cont}$ abhängen, welcher die Ähnlichkeit zwischen dem Wahrscheinlichkeitsdatensatz GPM_x und der projizierten Segmentierung misst. Eine mögliche Wahl für diesen Term ist

$$K_{segm}(x, m) = 1 - 2 \cdot \frac{GPM(x) \cdot Pr(m)}{GPM(x) \cdot GPM(x) + Pr(m) \cdot Pr(m)}$$

wobei GPM(x) den zum dreidimensionalen Trainingsmammographiedatensatz x gehörenden Wahrscheinlichkeitsdatensatz bezeichnet, und wobei das Produkt pixelweise zu verstehen ist. Auch dieser Term kann jeweils über mehrere Trainingsbilder gemittelt werden:

$$K_{segm}(x_1, \ldots, x_N, m_1, \ldots, m_N) = 1 - \frac{2}{N} \cdot \sum_{n=1}^{N} \frac{GPM(x_n) \cdot Pr(m_n)}{GPM(x_n) \cdot GPM(x_n) + Pr(m_n) \cdot Pr(m_n)}$$

Dieser Beitrag basiert auf dem Sørensen-Dice Koeffizienten. Alternativ kann dieser Beitrag aber auch beispielsweise auf dem Jaccard-Index oder dem Tversky-Index basieren.

In diesem Ausführungsbeispiel werden die Parameter Generatorfunktion G und der Klassifikatorfunktion C durch Minimierung der Kostenfunktionen $K_G$ und $K_C$ mittels Rückpropagation angepasst. Hierbei ist dem Fachmann die Rückpropagation für künstliche neuronale Netzwerke bekannt, auf eine genauere Beschreibung wird daher hier verzichtet.

Der letzte Schritt des dargestellten Ausführungsbeispiels ist das Bereitstellen PROV der Generatorfunktion G mittels der Schnittstelle DS.1.

| Tabelle B: Pseudocode zum Bereitstellen einer Generatorfunktion | |
|---|---|
| B.1 | G.init(G_params_initial) |
| B.2 | C.init(C_params_initial) |
| B.3 | a = 0.01 |
| B.4 | func train(network G, network C, image_3d x, image_3d m, image_2d y): |
| B.5 | image_2d G_x = G.convert(x) |
| B.6 | float C_y = C.apply_to(y) |
| B.7 | C.params -= a*back_prop(C, C.loss(1, C_y)) |
| B.8 | float C_G_x = C.apply_to(G_x) |

(fortgesetzt)

| Tabelle B: Pseudocode zum Bereitstellen einer Generatorfunktion | |
|---|---|
| B.9 | C.params -= a*back_prop(C, C.loss(0, C_G_x)) |
| B.10 | C_G_x = C.apply_to(G_x) |
| B.11 | G.params -= a*back_prop(G, G.loss(C_G_x, m, x)) |

Tabelle B zeigt Pseudocode für das in Fig. 3 und Fig. 4 dargestellte Ausführungsbeispiel zum Bestimmen einer Generatorfunktion G. In den Codezeilen B.1 und B.2 werden die Generatorfunktion G und die Klassifikatorfunktion C als künstliche neuronale Netzwerke mit Standardparametern initialisiert. Die Lerngeschwindigkeit "a" quantifiziert die Geschwindigkeit des Lernprozesses und wird in Zeile B.3 initialisiert.

In Codezeile B.4 wird die Funktion "train" deklariert, welche einen Trainingsschritt auf die Generatorfunktion G und die Klassifikatorfunktion C anwendet, der auf einem dreidimensionalen Trainingsmammographiedatensatz x und einem realen zweidimensionalen Trainingsmammographiedatensatz y basiert. Der Funktionsaufruf dieser Funktion implementiert die Schritte des ersten Trainings-Empfangen TREC-1 und des zweiten Trainings-Empfangen TREC-2.

In Codezeile B.5 wird ein synthetischer zweidimensionaler Mammographiedatensatz G_x durch Anwendung der Generatorfunktion G auf den dreidimensionalen Trainingsmammographiedatensatz x erzeugt. Dies entspricht dem Verfahrensschritt des ersten Trainings-Bestimmens TDET-1.

In Codezeilen B.6 wird ein zweiter Wahrscheinlichkeitswert C_y durch Anwendung der Klassifikatorfunktion C auf den realen zweidimensionalen Trainingsmammographiedatensatz y bestimmt. Dies entspricht dem Verfahrensschritt des dritten Trainings-Bestimmens TDET-3. In Codezeile B.7 werden die Parameter der Klassifikatorfunktion C angepasst, indem die Kostenfunktion mittels Rückpropagation minimiert wird. Die Kostenfunktion entspricht hierbei der binären Kreuzentropie. Diese Codezeile entspricht einem Teil des Verfahrensschritts des Anpassens ADJ.

In Codezeilen B.8 wird ein erster Wahrscheinlichkeitswert C_G_x durch Anwendung der Klassifikatorfunktion C auf den synthetischen zweidimensionalen Mammographiedatensatz G_x bestimmt. Dies entspricht dem Verfahrensschritt des zweiten Trainings-Bestimmens TDET-3. In Codezeile B.9 werden die Parameter der Klassifikatorfunktion C angepasst, indem die Kostenfunktion mittels Rückpropagation minimiert wird. Die Kostenfunktion entspricht hierbei der binären Kreuzentropie. Diese Codezeile entspricht einem Teil des Verfahrensschritts des Anpassens ADJ.

In den Codezeilen B.10 und B.11 wird erneut der erste Wahrscheinlichkeitswert C_G_x durch Anwendung der Klassifikatorfunktion C auf den synthetischen zweidimensionalen Mammographiedatensatz G_x bestimmt. Da in Codezeile B.9 die Parameter der Klassifikatorfunktion C angepasst wurden, ändert sich der Zahlenwert des ersten Wahrscheinlichkeitswertes. In Codezeile B.11 werden die Parameter der Generatorfunktion G angepasst, indem die Kostenfunktion der Generatorfunktion G mittels Rückpropagation minimiert wird. Die Kostenfunktion entspricht hierbei der bereits definierten Funktion $K_G$. Diese Codezeile entspricht einem Teil des Verfahrensschritts des Anpassens ADJ.

Fig. 5 zeigt ein erstes Ausführungsbeispiel einer Generatorfunktion G, welche als künstliches neuronales Netzwerk ausgebildet ist. In diesem Ausführungsbeispiel umfasst die Generatorfunktion ein erstes Teilnetzwerk G.3D, ein zweites Teilnetzwerk G.2D, sowie eine Projektionsschicht G.PL. Das erste Teilnetzwerk bildet einen dreidimensionalen Mammographiedatensatz x, x' auf einen ersten Merkmalsvektor FV.3D ab. Die Projektionsschicht G.PL bildet den ersten Merkmalsvektor FV.3D auf einen zweiten Merkmalsvektor FV.2D ab. Das zweite Teilnetzwerk G.2D bildet den zweiten Merkmalsvektor FV.2D auf einen synthetischen zweidimensionalen Mammographiedatensatz G_x, G_x' ab.

Der Eingabewert der Generatorfunktion G ist hierbei entweder ein erster zweidimensionaler Mammographiedatensatz x, wenn der optionale Schritt des Entfernens vorher nicht auf den ersten zweidimensionaler Mammographiedatensatz x angewendet wurde, oder ein zweiter Mammographiedatensatz x', wenn der optionale Schritt des Entfernens vorher auf den ersten zweidimensionaler Mammographiedatensatz x angewendet wurde.

Sowohl das erste Teilnetzwerk G.3D als auch das zweite Teilnetzwerk G.2D sind in diesem Ausführungsbeispiel faltende neuronale Netzwerke (ein englischer Fachbegriff ist "convolutional neural network").

Das erste Teilnetzwerk G.3D umfasst in diesem Ausführungsbeispiel mehrere dreidimensionale Faltungsschichten CL.3D, das zweite Teilnetzwerk G.2D umfasst in diesem Ausführungsbeispiel mehrere zweidimensionale Faltungsschichten CL.2D. Das erste Teilnetzwerk G.3D umfasst in diesem Ausführungsbeispiel weiterhin zumindest eine Bündelungsschicht, das zweite Teilnetzwerk G.2D umfasst in diesem Ausführungsbeispiel weiterhin zumindest eine Entbündelungsschicht.

Vorteilhafterweise umfasst das erste Teilnetzwerk G.3D und/oder das zweite Teilnetzwerk G.2D ein oder mehrere Einsatzmodule (ein englischer Fachbegriff ist "inception module"). Ein Einsatzmodul kombiniert zumindest zwei Faltungsoperatoren mit unterschiedlich großen Faltungsmasken zu einem gemeinsamen Ausgabewert. Die Verwendung von Einsatzmodulen ist beispielsweise aus der Netzwerkarchitektur "GoogLeNet" bekannt, hierdurch müssen die Größen der Faltungsmasken in den jeweiligen Schichten nicht vorgegeben werden, sondern können im Lernprozess aus mehreren Möglichkeiten automatisch ausgewählt werden. Weiterhin kann das erste Teilnetzwerk G.3D, das zweite Teilnetzwerk G.2D Residualverbindungen (ein englischer Fachbegriff ist "residual connections") umfassen.

Im dargestellten Ausführungsbeispiel ist der zweidimensionale Mammographiedatensatz G_x, G_x' bezüglich einer ersten Richtung u und bezüglich einer zweiten Richtung v ausgedehnt, wobei der dreidimensionale Mammographiedatensatz x, x' weiterhin bezüglich einer dritten Richtung w ausgedehnt ist. Die Projektionsschicht G.PL ist im dargestellten Ausführungsbeispiel ein dreidimensionaler Faltungsoperator bzw. eine dreidimensionale Faltungsschicht CL.3D, wobei die Ausdehnung des Faltungskerns bezüglich der dritten Richtung w auf der Ausdehnung $L_3$ des dreidimensionalen Mammographiedatensatzes x, x' bezüglich der dritten Richtung w basiert. Insbesondere ist der Faltungsoperator so gewählt, dass der Ausgabewert bezüglich der dritten Richtung w nur eine Ausdehnung von einem Voxel aufweist, insbesondere also einen dreidimensionalen Wert in einen zweidimensionalen Wert abbildet. Insbesondere ist die Ausdehnung des Faltungskerns bezüglich der dritten Richtung w identisch mit der Ausdehnung des Eingabewertes des Faltungsoperators bezüglich der dritten Richtung w, diese entspricht der Ausdehnung $L_3$ des dritten Mammographiedatensatzes x, x', welche basierend auf den Auswirkungen der Bündelungs- und Entbündelungsschichten des ersten Teilnetzwerkes G.3D auf die Ausdehnung des Datensatzes bezüglich der dritten Richtung w korrigiert wurde.

Fig. 6 zeigt ein zweites Ausführungsbeispiel einer Generatorfunktion G. In diesem Ausführungsbeispiel umfasst die Generatorfunktion ein erstes Teilnetzwerk G.3D, ein zweites Teilnetzwerk G.2D, eine Projektionsschicht G.PL, sowie ein drittes Teilnetzwerk PM.3D. Das erste Teilnetzwerk bildet einen dreidimensionalen Mammographiedatensatz x, x' auf einen ersten Merkmalsvektor FV.3D ab. Die Projektionsschicht G.PL bildet den ersten Merkmalsvektor FV.3D auf einen zweiten Merkmalsvektor FV.2D ab. Das zweite Teilnetzwerk G.2D bildet den zweiten Merkmalsvektor FV.2D auf einen synthetischen zweidimensionalen Mammographiedatensatz G_x, G_x' ab. Das dritte Teilnetzwerk G.PM2D bildet den zweiten Merkmalsvektor FV.2D auf eine Wahrscheinlichkeitsdatensatz GPM_x, GPM_x' ab, wobei der Wahrscheinlichkeitsdatensatz GPM_x, GPM_x' jedem Pixel des synthetischen zweidimensionalen Mammographiedatensatzes G_x, G_x' einen Wahrscheinlichkeitswert zwischen 0 und 1 zuordnet. In anderen Worten kann der Wahrscheinlichkeitsdatensatz GPM_x, GPM_x' daher auch als Bilddatensatz aufgefasst werden.

Das erste Teilnetzwerk G.3D und das zweite Teilnetzwerk G.2D sowie die Projektionsschicht G.PL können die in der Beschreibung zur Fig. 5 beschriebenen Merkmale und vorteilhaften Weiterbildungen aufweisen.

Das dritte Teilnetzwerk G.PM2D weist in diesem Ausführungsbeispiel eine oder mehrere zweidimensionale Faltungsschichten CL.2D auf. Vorteilhafterweise kann das dritte Teilnetzwerk G.PM2D Bündelungsschichten und Entbündelungsschichte aufweisen. Weiterhin kann das dritte Teilnetzwerk G.PM2D Residualverbindungen umfassen.

In diesem Ausführungsbeispiel ist der Ausgabewert der Generatorfunktion G ein Paar umfassend einen synthetischen zweidimensionalen Mammographiedatensatz G_x, G_x' sowie einen zweidimensionalen Wahrscheinlichkeitsdatensatz GPM_x, GPM_x'. Die Notation G(x), G(x') bezeichnet aber auch in diesem Fall nur den synthetischen zweidimensionalen Mammographiedatensatz G_x, G_x'.

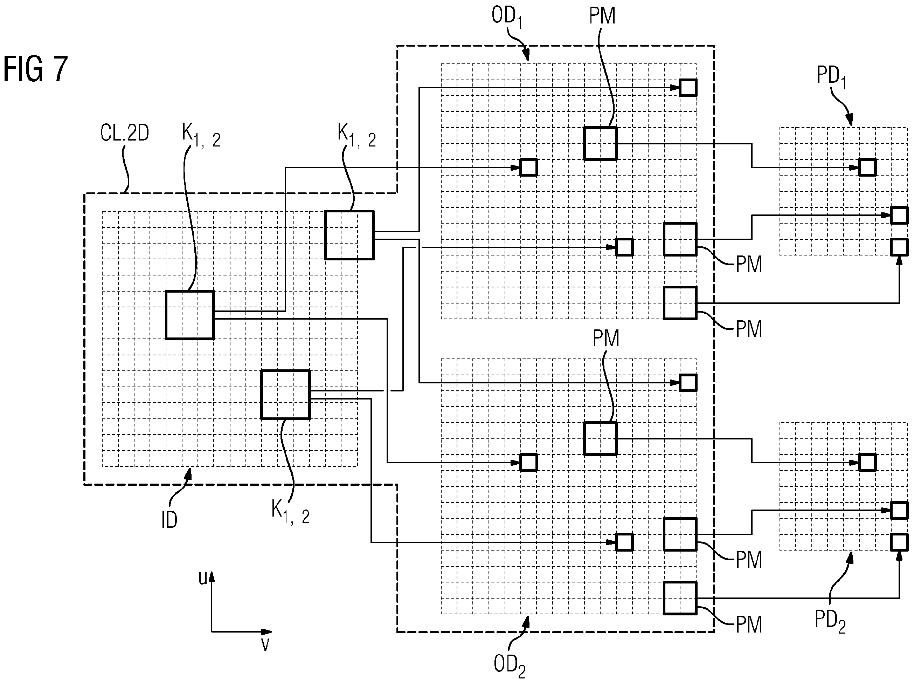

Fig. 7 zeigt eine zweidimensionale faltende Schicht CL.2D und eine Bündelungsschicht eines faltenden neuronalen Netzwerkes, insbesondere des zweiten Teilnetzwerkes G.2D. Die dargestellte zweidimensionale faltende Schicht CL.2D realisiert einen zweidimensionalen Faltungsoperator.

Die zweidimensionale faltende Schicht bildet hierbei ein zweidimensionales Eingabearray (welches in diesem Ausführungsbeispiel ein Eingabebild ist, welches 8x8 Pixel aufweist) auf zwei zweidimensionale Ausgabearrays ab, wobei die Ausdehnung des zweidimensionalen Eingabearrays und jedes der zweidimensionalen Ausgabearrays bezüglich jeder Dimension identisch ist. Das erste Ausgabearray ist eine Faltung des Eingabearrays mit einem ersten Kern $K_1$, das zweite Ausgabearray ist eine Faltung des Eingabearrays mit einem zweiten Kern $K_2$:

$$(\mathrm{ID}\ast K_{1,2})[n_1,n_2] = \sum_{n_1'=1}^{N_1}\sum_{n_2'=1}^{N_2}\mathrm{ID}[n_1',n_2']\cdot K_{1,2}[n_1\text{-}n_1',n_2\text{-}n_2']$$

Hierbei bezeichnet $\mathrm{ID}[n_1, n_2]$ bzw. $K_{1,2}[n_1, n_2]$ den Eintrag des Eingabearrays bzw. des Kerns mit den Indizes bzw. Koordinaten $n_1, n_2$. bzw. des Kernes $K_1, K_2$ an den Indizes (Koordinaten) $n_1$ und $n_2$. Hierbei ist $\mathrm{ID}[n_1, n_2] = 0$ bzw. $K_{1,2}[n_1, n_2] = 0$, falls einer der beiden Indizes außerhalb des gültigen Bereichs liegt. Alternativ können auch andere Randbedingungen bzw. Randwerte (beispielsweise periodische Randbedingungen bzw. Randwerte) verwendet werden. $N_1$ ist die Ausdehnung des Eingabebildes in die erste Richtung u, $N_2$ ist die Ausdehnung des Eingabebildes in die zweite Richtung v, jeweils gemessen in einer Anzahl von Pixeln. Das Ergebnis wird mit $DL_{1,2} := \mathrm{ID}\ast K_{1,2}$ bezeichnet.

Eine Faltung kann auf gleiche Weise in beliebigen Dimensionen definiert werden, also d-dimensionale Eingabebilder auf d-dimensionale Ausgabebilder abbilden, insbesondere in drei Dimensionen:

$$(ID*K_{1,2})[n_1, n_2, n_3] =$$

$$= \sum_{n_1' = 1}^{N_1} \sum_{n_2' = 1}^{N_2} \sum_{n_3' = 1}^{N_3} ID[n_1', n_2', n_3'] \cdot K_{1,2}[n_1-n_1', n_2-n_2', n_3-n_3']$$

Der Kern $K_1$, $K_2$ ist hierbei durch eine Kernmaske und durch Kernwerte definiert. Hierbei ist die Kernmaske die Ausdehnung des Kerns (die gültigen Indizes), und die Kernwerte sind die tatsächlichen Werte des Kerns. Die Kernmaske kann insbesondere durch die Größe in Pixeln und/oder Voxeln angegeben werden, die Kernwerte in Form eines Arrays. Beispielsweise kann als Kern mit der Ausdehnung 3x3 Pixel

$$K = \begin{pmatrix} -1 & 1 & -1 \\ 1 & 1 & 1 \\ -1 & 1 & -1 \end{pmatrix} = \begin{pmatrix} K[-1,-1] & K[-1,0] & K[-1,1] \\ K[0,-1] & K[0,0] & K[0,1] \\ K[1,-1] & K[1,0] & K[1,1] \end{pmatrix}$$

verwendet werden. Es sind auch möglich, nicht-rechteckige bzw. nicht-quaderförmige Kernmasken zu verwenden. Diese können aber analog zu rechteckigen bzw. quaderförmigen Kernmasken behandelt werden, wenn alle Kernwerte außerhalb der Kernmaske mit 0 belegt werden.

Im dargestellten Ausführungsbeispiel wird der Faltungsoperator innerhalb eines künstlichen neuronalen Netzwerkes realisiert. Das Eingabearray entspricht hierbei einer ersten Schicht des künstlichen neuronalen Netzwerkes, und die einen oder mehreren Ausgabearrays entsprechen einer zweiten Schicht des künstlichen neuronalen Netzwerkes, wobei die erste und die zweite Schicht benachbarte Schichten im künstlichen neuronalen Netzwerk sind. Die Kernmaske ist durch die Topologie (Struktur der Kanten und Knoten) des künstlichen neuronalen Netzwerkes gegeben, die Kernwerte durch die Kantengewichte. Durch das Training des künstlichen neuronalen Netzwerkes werden also in diesem Ausführungsbeispiel nur die Kernwerte verändert, nicht die Kernmaske. Beim Training werden hierfür Kantengewichte zur jeweils gleichen Eintrag in der Kernmaske nur simultan geändert, um zu erreichen, dass der Kern bzw. die Kernwerte für jeden Eintrag des Ausgabearrays gleich sind.

Das dargestellte Ausführungsbeispiel umfasst neben dem zweidimensionalen Faltungsoperator CL.2D auch einen zweidimensionalen Bündelungsoperator (ein englischer Fachbegriff für "Bündelung" ist "pooling") in Form einer Bündelungsschicht. Dieser bildet ein zweidimensionales Eingabearray $OD_{1,2}$ auf ein zweidimensionales Ausgabearray $PD_{1,2}$ ab, wobei die Ausdehnung des zweidimensionalen Ausgabearrays $PD_{1,2}$ bezüglich mindestens einer ersten Dimension kleiner ist als die Ausdehnung des zweidimensionalen Eingabearrays $OD_{1,2}$ bezüglich dieser ersten Dimension. Im dargestellten Ausführungsbeispiel wird eine Maximalwertbündelung von jeweils 2x2 Pixeln verwendet, das Ausgabearray $PD_{1,2}$ ergibt sich also aus

$$PD_{1,2}[n_1,n_2] = \max\left(\left\{ \begin{matrix} OD_{1,2}[2n_1, 2n_2], & OD_{1,2}[2n_1, 2n_2+1], \\ OD_{1,2}[2n_1+1, 2n_2], & OD_{1,2}[2n_1+1, 2n_2+1] \end{matrix} \right\}\right)$$

Der zu bündelnde Bereich (hier jeweils ein Bereich von 2x2 Pixeln) wird auch als Bündelungsmaske PM (ein englischer Begriff ist "pooling mask") bezeichnet.

Das erste Teilnetzwerk und/oder das zweite Teilnetzwerk können auch einen Entbündelungsoperator (ein englischer Fachbegriff ist "unpooling operator"). Dieser bildet ein Eingabearray auf ein Ausgabearray ab, wobei die Ausdehnung des Ausgabearrays bezüglich mindestens einer Dimension größer ist als die Ausdehnung des Eingabearrays. Im zweidimensionalen Fall kann beispielsweise die Anzahl der Pixel vervierfacht werden, indem aus einem Pixel des Eingabearrays 2x2 Pixel des Ausgabearrays erzeugt werden. Beispielsweise kann jeder der 2x2 Pixel den Wert des einen Pixels des Eingabearrays erhalten, alternativ kann nur ein Pixel der 2x2 Pixel des Ausgabearrays den Wert des einen Pixels des Eingabearrays erhalten und die anderen Pixel mit dem Wert 0 belegt werden.

Sowohl ein Bündelungsoperator als auch ein Entbündelungsoperator können, wie im dargestellten Ausführungsbeispiel, durch ein künstliches neuronales Netzwerk realisiert werden.

Fig. 8 zeigt die Datenstruktur beim Entfernen RMV einer zweidimensionalen Schicht w.1, w.2, w.7, w.8 des dreidimensionalen Mammographiedatensatzes x mittels der Recheneinheit. In diesem Ausführungsbeispiel wird das Entfernen RMV mehrfach ausgeführt, und erzeugt aus dem ersten dreidimensionalen Mammographiedatensatz x einen zweiten dreidimensionalen Mammographiedatensatz x', wobei der zweite dreidimensionale Mammographiedatensatz x' weniger Voxel umfasst als der erste dreidimensionale Mammographiedatensatz x.

Sowohl der erste als auch der zweite dreidimensionale Mammographiedatensatz x, x' sind in eine erste Richtung u, in eine zweite Richtung v und eine dritte Richtung w ausgedehnt, wobei die zweite Richtung v orthogonal zur ersten Richtung u ist, und wobei die dritte Richtung w orthogonal zur ersten Richtung und zur zweiten Richtung v ist.

Die Ausdehnung des ersten und des zweiten dreidimensionalen Mammographiedatensatzes x, x' in die erste Richtung u ist $L_1$ Voxel, die Ausdehnung des ersten und des zweiten dreidimensionalen Mammographiedatensatzes x, x' in die zweite Richtung v ist $L_2$ Voxel. Die Ausdehnung des ersten dreidimensionalen Mammographiedatensatzes x in die dritte Richtung w ist $L_3$ Voxel, die Ausdehnung des zweiten dreidimensionalen Mammographiedatensatzes x' in die dritte Richtung w ist $L_3'$ Voxel, wobei $L_3' < L_3$. In diesem Ausführungsbeispiel ist $L_1 = L_2 = L_3 = 8$, und $L_3' = 4$. In diesem Ausführungsbeispiel wird der Verfahrensschritt Entfernen RMV daher viermal ausgeführt. Natürlich ist es auch möglich, andere Ausdehnungen des ersten und des zweiten dreidimensionalen Mammographiedatensatz x, x' zu verwenden.

Der erste dreidimensionale Mammographiedatensatz x umfasst in diesem Ausführungsbeispiel acht zweidimensionale Schichten w.1, ..., w.8, der zweite dreidimensionale Mammographiedatensatz x' umfasst in diesem Ausführungsbeispiel vier zweidimensionale Schichten w.3, ..., w.6. Jede dieser zweidimensionalen Schichten w.1, ..., w.8 ist in die erste Richtung u und in die zweite Richtung v ausgedehnt und umfasst $L_1 \cdot L_2$ Voxel bzw. Pixel. Es wird also in der viermaligen Wiederholung des Verfahrensschrittes Entfernen RMV die zweidimensionalen Schichten w.1, w.2, w.7 und w.8 entfernt.

Die zu entfernenden Schichten w.1, w.2, w.7, w.8 werden in diesem Ausführungsbeispiel basierend auf der Position einer Brustwarze im ersten dreidimensionalen Mammographiedatensatz bestimmt x. Insbesondere wird die zweidimensionale Brustwarzenschicht w.B aus den zweidimensionalen Schichten w.1, ..., w.8 des ersten dreidimensionalen Mammographiedatensatzes x bestimmt, welche die Position der Brustwarze umfasst. Das Bestimmen der zweidimensionalen Brustwarzenschicht w.B erfolgt hierbei durch ein weiteres faltendes neuronales Netzwerk, welches als Eingabedaten einen dreidimensionalen Mammographiedatensatz x erhält und als Ausgabewert die Koordinate der zweidimensionalen Brustwarzenschicht w.B erzeugt. Dieser weitere faltende neuronale Netzwerk wird mittels einer Mehrzahl an Trainingspaaren trainiert, wobei jedes der Trainingspaare einen dreidimensionalen Trainingsmammographiedatensatz x.1, ..., x.S sowie eine zugehörige Trainingsposition bzw. Trainingsschicht umfasst, wobei die Trainingsposition bzw. Trainingsschicht der Position bzw. der Schicht der Brustwarze im dreidimensionalen Trainingsmammographiedatensatz x.1, ..., x.S entspricht und durch einen Anwender bestimmt wurde.

Alternativ können die zu entfernenden Schichten w.1, w.2, w.7, w.8 basierend auf Gewichtungsfaktoren von Voxeln des dreidimensionalen Mammographiedatensatzes x bestimmt werden. Die Gewichtungsfaktoren können insbesondere einem Wahrscheinlichkeitswert entsprechen, dass der zugehörige Voxel eine Läsion abbildet. Die Gewichtungsfaktoren für die Voxel des dreidimensionalen Mammographiedatensatzes x können insbesondere durch ein weiteres faltendes neuronales Netzwerk bestimmt werden, welches als Eingabedaten einen dreidimensionalen Mammographiedatensatz x erhält und als Ausgabewert einen dreidimensionalen Gewichtungsdatensatz erzeugt, welcher jedem der Voxel des dreidimensionalen Mammographiedatensatzes x den Wahrscheinlichkeitswert zuweisen, dass der Voxel eine Läsion abbildet. Das weitere faltende neuronale Netwerk kann insbesondere basierend auf Trainingspaaren trainiert werden, wobei jedes Trainingspaar einen dreidimensionalen Trainingsmammographiedatensatz x.1, ..., x.S sowie eine zugehörige Trainingssegmentierung umfasst, wobei die Trainingssegmentierung durch einen Anwender vorgenommen wurde, welcher Läsionen in den dreidimensionalen Trainingsmammographiedatensätzen x.1, ..., x.S segmentiert. Die zu entfernenden Schichten w.1, w.2, w.7, w.8 basieren in dem dargestellten Ausführungsbeispiel auf der zweidimensionalen Gewichtungsschicht w.G, wobei die zweidimensionale Gewichtungsschicht w.G die zweidimensionale Schicht w.1, ..., w.8 ist, welche den Voxel umfasst, denen der maximale Gewichtungsfaktor zugeordnet ist.

In diesem Ausführungsbeispiel ist sowohl die zweidimensionale Brustschicht w.B als auch die zweidimensionale Gewichtungsschicht w.G identisch mit der zweidimensionalen Schicht w.4. In beiden Alternativen werden die zu entfernenden zweidimensionalen Schichten w.1, w.2, w.7, w.8 derart bestimmt, dass der resultierende zweite dreidimensionale Mammographiedatensatz x' eine festgelegte Ausdehnung $L_3'$ in die dritte Richtung w aufweist, und dass die zweidimensionale Brustschicht w.B bzw. die zweidimensionale Gewichtungsschicht w.G die zentrale Schicht (bei ungeradem $L_3'$) oder eine der beiden zentralen Schichten (bei geradem $L_3'$) ist.

## Patentansprüche

1.  Verfahren zum Bestimmen eines zweidimensionalen Mammographiedatensatzes (G_x, G_x'), umfassend die folgenden Verfahrensschritte:

    - Empfangen (REC) eines dreidimensionalen Mammographiedatensatzes (x, x') eines Untersuchungsbereiches mittels einer Schnittstelle (DS.1),
    - Erstes Bestimmen (DET-1) eines zweidimensionalen Mammographiedatensatzes (G_x, G_x') des Untersuchungsbereiches durch Anwendung einer trainierten Generatorfunktion (G) auf den dreidimensionalen Mammographiedatensatz (x') mittels einer Recheneinheit (DS.2),

wobei die trainierte Generatorfunktion (G) auf einem trainierten GA-Algorithmus (GA) basiert.

2. Verfahren nach dem Anspruch 1, wobei der trainierte GA-Algorithmus eine generative Funktion (G) und ein klassifizierende Funktion (C) umfasst,
und wobei die trainierte Generatorfunktion (G) mit dem generativen Teilnetzwerk (G) des trainierten GA-Algorithmus identisch ist.

3. Verfahren nach einem der vorstehenden Ansprüche, wobei die trainierte Generatorfunktion (G) ein künstliches neuronales Netzwerk ist.

4. Verfahren nach einem der vorstehenden Ansprüche, wobei die trainierte Generatorfunktion (G) eine Verkettung einer ersten Teilfunktion (G.3D), einer ersten Projektionsfunktion (G.PL) und einer zweiten Teilfunktion (G.2D) umfasst,
wobei die erste Teilfunktion (G.3D) den dreidimensionalen Mammographiedatensatz (x, x') auf einen ersten Merkmalsvektor abbildet (FV.3D),
wobei die Projektionsfunktion (G.PL) den ersten Merkmalsvektor (FV.3D) auf den zweiten Merkmalsvektor (FV.2D) abbildet, und wobei die zweite Teilfunktion (G.2D) den zweiten Merkmalsvektor (FV.2D) auf den zweidimensionalen Mammographiedatensatz (G_x, G_x') abbildet.

5. Verfahren nach dem Anspruch 4, wobei die erste Teilfunktion (G.3D) wenigstens einen dreidimensionalen Faltungsoperator (CL.3D) umfasst.

6. Verfahren nach dem Anspruch 4 oder 5, wobei die zweite Teilfunktion (G.2D) wenigstens einen zweidimensionalen Faltungsoperator (CL.2D) umfasst.

7. Verfahren nach einem der Ansprüche 4 bis 6, wobei die Projektionsfunktion (G.PL) ein dreidimensionaler Faltungsoperator ist,
wobei der zweidimensionale Mammographiedatensatz (G_x, G_x') bezüglich einer ersten Richtung (u) und bezüglich einer zweiten Richtung (v) ausgedehnt ist,
wobei der dreidimensionale Mammographiedatensatz (x, x') weiterhin bezüglich einer dritten Richtung (w) ausgedehnt ist, und wobei die Ausdehnung des Faltungskerns des dreidimensionalen Faltungsoperators bezüglich der dritten Richtung (w) identisch mit der um Auswirkungen eines Bündelungsoperators und/oder eines Entbündelungsoperators der ersten Teilfunktion (G.3D) korrigierten Ausdehnung des dreidimensionalen Mammographiedatensatzes (x, x') bezüglich der dritten Richtung (w) ist.

8. Verfahren nach einem der vorstehenden Ansprüche, wobei der zweidimensionale Mammographiedatensatz (G_x, G_x') bezüglich einer ersten Richtung (u) und bezüglich einer zweiten Richtung ausgedehnt (v) ist, weiterhin umfassend den folgenden Verfahrensschritt:

- Entfernen (RMV) einer zweidimensionalen Schicht (w.1, w.2, w7, w.8) des dreidimensionalen Mammographiedatensatzes (x) mittels der Recheneinheit (DS.2), wobei die zweidimensionale Schicht (w.1, w.2, w7, w.8) bezüglich der ersten Richtung (u) und der zweiten Richtung (v) ausgedehnt ist.

9. Verfahren nach dem Anspruch 8, wobei die zweidimensionale Schicht (w.1, w.2, w7, w.8) basierend auf der Position der Brustwarze im dreidimensionalen Mammographiedatensatz (x) bestimmt wird.

10. Verfahren nach dem Anspruch 8, wobei die zweidimensionale Schicht (w.1, w.2, w7, w.8) basierend auf Gewichtungsfaktoren von Voxeln des dreidimensionalen Mammographiedatensatzes (x) bestimmt wird,
wobei ein Gewichtungsfaktor eines Voxels der Wahrscheinlichkeit entspricht, dass der Voxel eine Läsion abbildet.

11. Verfahren nach einem der vorstehenden Ansprüche, wobei der zweidimensionale Mammographiedatensatz (G_x, G_x') ein oder mehrere Pixel umfasst, weiterhin umfassend den folgenden Verfahrensschritt:

- Zweites Bestimmen (DET-2) eines zweidimensionalen Wahrscheinlichkeitsdatensatzes (GPM_x, GPM_x') mittels der Recheneinheit (DS.2),
wobei der zweidimensionale Wahrscheinlichkeitsdatensatz (GPM_x, GPM_x') jedem der ein oder mehreren Pixel des zweidimensionalen Mammographiedatensatzes (G_x, G_x') eine Wahrscheinlichkeit zuordnet, dass der jeweilige Pixel eine Läsion abbildet.

12. Bestimmungssystem (DS), umfassend die folgenden Einheiten:

   - Schnittstelle (DS.1), ausgebildet zum Empfangen (REC) eines dreidimensionalen Mammographiedatensatzes (x, x') eines Untersuchungsbereiches,
   - Recheneinheit (DS.2), ausgebildet zum ersten Bestimmen (DET-1) eines zweidimensionalen Mammographiedatensatzes (G_x, G_x') des Untersuchungsbereiches durch Anwendung einer trainierten Generatorfunktion (G) auf den dreidimensionalen Mammographiedatensatz (x, x'),
   wobei die trainierte Generatorfunktion (G) auf einem trainierten GA-Netzwerk (GA) basiert.

13. Bestimmungssystem (DS) nach dem Anspruch 12, weiterhin ausgebildet ein Verfahren nach den Ansprüchen 2 bis 11 auszuführen.

14. Computerprogrammprodukt mit einem Computerprogramm, welches direkt in eine Speichereinheit (DS.3) eines Bestimmungssystems (DS) ladbar ist, mit Programmabschnitten, um alle Schritte des Verfahrens nach einem der Ansprüche 1 bis 11 auszuführen, wenn die Programmabschnitte von dem Bestimmungssystem (DS) ausgeführt werden.

15. Computerlesbares Speichermedium, auf welchem von einem Bestimmungssystem (DS) lesbare und ausführbare Programmabschnitte gespeichert sind, um alle Schritte des Verfahrens nach einem der Ansprüche 1 bis 11 auszuführen, wenn die Programmabschnitte von dem Bestimmungssystem (DS) ausgeführt werden.

**Claims**

1. Method for determining a two-dimensional mammography dataset (G_x, G_x'), comprising the following method steps:

   - Receipt (REC) of a three-dimensional mammography dataset (x, x') of an examination region by means of an interface (DS.1),
   - First determination (DET-1) of a two-dimensional mammography dataset (G_x, G_x') of the examination region by application of a trained generator function (G) to the three-dimensional mammography dataset (x') by means of a processing unit (DS.2),
   wherein the trained generator function (G) is based on a trained GA algorithm (GA).

2. Method according to claim 1, wherein the trained GA algorithm comprises a generative function (G) and a classifying function (C), and wherein the trained generator function (G) is identical to the generative subnetwork (G) of the trained GA algorithm.

3. Method according to one of the preceding claims, wherein the trained generator function (G) is an artificial neural network.

4. Method according to one of the preceding claims, wherein the trained generator function (G) comprises a concatenation of a first subfunction (G.3D), a first projection function (G.PL) and a second subfunction (G.2D), wherein the first subfunction (G.3D) maps the three-dimensional mammography dataset (x, x') to a first feature vector (FV.3D), wherein the projection function (G.PL) maps the first feature vector (FV.3D) to the second feature vector (FV.2D), and wherein the second subfunction (G.2D) maps the second feature vector (FV.2D) to the two-dimensional mammography dataset (G_x, G_x').

5. Method according to claim 4, wherein the first subfunction (G.3D) comprises at least one three-dimensional convolutional operator (CL.3D).

6. Method according to claim 4 or 5, wherein the second subfunction (G.2D) comprises at least one two-dimensional convolutional operator (CL.2D).

7. Method according to one of claims 4 to 6, wherein the projection function (G.PL) is a three-dimensional convolutional operator,

wherein the two-dimensional mammography dataset (G_x, G_x') is extended in relation to a first direction (u) and in relation to a second direction (v),

wherein the three-dimensional mammography dataset (x, x') is furthermore extended in relation to a third direction (w), and wherein the extent of the convolution kernel of the three-dimensional convolutional operator in relation to the third direction (w) is identical to the extent of the three-dimensional mammography dataset (x, x'), which has been corrected based on the effects of a pooling operator and/or an unpooling operator of the first subfunction (G.3D), in relation to the third direction (w).

8. Method according to one of the preceding claims, wherein the two-dimensional mammography dataset (G_x, G_x') is extended in relation to a first direction (u) and in relation to a second direction (v), furthermore comprising the following method step:

- Removal (RMV) of a two-dimensional layer (w.1, w.2, w7, w.8) of the three-dimensional mammography dataset (x) by means of the processing unit (DS.2), wherein the two-dimensional layer (w.1, w.2, w7, w.8) is extended in relation to the first direction (u) and the second direction (v).

9. Method according to claim 8, wherein the two-dimensional layer (w.1, w.2, w7, w.8) is determined based on the position of the nipple in the three-dimensional mammography dataset (x) .

10. Method according to claim 8, wherein the two-dimensional layer (w.1, w.2, w7, w.8) is determined based on weighting factors of voxels of the three-dimensional mammography dataset (x),

wherein a weighting factor of a voxel corresponds to the probability that the voxel is mapping a lesion.

11. Method according to one of the preceding claims, wherein the two-dimensional mammography dataset (G_x, G_x') comprises one or more pixels, further comprising the following method step:

- Second determination (DET-2) of a two-dimensional probability dataset (GPM_x, GPM_x') by means of the processing unit (DS.2),

wherein the two-dimensional probability dataset (GPM_x, GPM_x') assigns to each of the one or more pixels of the two-dimensional mammography dataset (G_x, G_x') a probability that the respective pixel is mapping a lesion.

12. Determination system (DS), comprising the following units:

- Interface (DS.1), embodied for receiving (REC) a three-dimensional mammography dataset (x, x') of an examination region,
- Processing unit (DS.2), embodied for first determination (DET-1) of a two-dimensional mammography dataset (G_x, G_x') of the examination region by application of a trained generator function (G) to the three-dimensional mammography dataset (x, x'),

wherein the trained generator function (G) is based on a trained GA network (GA).

13. Determination system (DS) according to claim 12, further embodied for carrying out a method according to claims 2 to 11.

14. Computer program product with a computer program, which is able to be loaded directly into a memory unit (DS.3) of a determination system (DS), with program sections for carrying out all steps of the method according to one of claims 1 to 11, when the program sections are executed by the determination system (DS).

15. Computer-readable storage medium, on which program sections able to be read and executed by a determination system (DS) are stored, for carrying out all steps of the method according to one of claims 1 to 11, when the program sections are executed by the determination system (DS).

**Revendications**

1. Procédé de détermination d'un ensemble (G_x, G_x') bidimensionnel de données de mammographie, comprenant les stades de procédé suivant :

- réception (REC) d'un ensemble (x, x') tridimensionnel de données de mammographie d'une partie à étudier au moyen d'une interface (DS.1),
- première détermination (DET-1) d'un ensemble (G_x, G_x') bidimensionnel de données de mammographie de la partie à étudier par application d'une fonction (G) de générateur ayant subi un apprentissage à l'ensemble (x') tridimensionnel de données de mammographie au moyen d'une unité (DS.2) de calcul, la fonction (G) de générateur ayant subi un apprentissage reposant sur un algorithme GA (GA) ayant subi un apprentissage.

2. Procédé suivant la revendication 1, dans lequel l'algorithme GA ayant subi un apprentissage comprend une fonction (G) générative et une fonction (C) classificatrice, et dans lequel la fonction (G) de générateur ayant subi un apprentissage est identique au réseau (G) partiel génératif de l'algorithme GA ayant subi un apprentissage.

3. Procédé suivant l'une des revendications précédentes, dans lequel la fonction (G) de générateur ayant subi un apprentissage est un réseau neuronal artificiel.

4. Procédé suivant l'une des revendications précédentes, dans lequel la fonction (G) de générateur ayant subi un apprentissage comprend un chaînage d'une première fonction (G.3D) partielle d'une première fonction (G.PL) de projection et d'une deuxième fonction (G.2D) partielle, la première fonction (G.3D) partielle reproduisant l'ensemble (x, x') tridimensionnel de données de mammographie sur un premier vecteur (FV.3D) de caractéristique, la fonction (G.PL) de projection reproduisant le premier vecteur (FV.3D) de caractéristique sur le deuxième vecteur (FV.2D) de caractéristique, et la deuxième fonction (G.2D) partielle reproduisant le deuxième vecteur (FV.2D) de caractéristique sur le deuxième ensemble (G_x, G_x') bidimensionnel de données de mammographie.

5. Procédé suivant la revendication 4, dans lequel la première fonction (G.3D) partielle comprend au moins un opérateur (CL.3D) de convolution tridimensionnel.

6. Procédé suivant la revendication 4 ou 5, dans lequel la deuxième fonction (G.2D) partielle comprend au moins un opérateur (CL.2D) de convolution bidimensionnel.

7. Procédé suivant l'une des revendications 4 à 6, dans lequel la fonction (G.PL) de projection est un opérateur de convolution tridimensionnel, dans lequel l'ensemble (G_x, G_x') bidimensionnel de données de mammographie est étendu dans une première direction (u) et dans une deuxième direction (v), l'ensemble (x, x') tridimensionnel de données de mammographie est étendu, en outre, dans une troisième direction (w), et l'étendue du noyau de convolution de l'opérateur de convolution tridimensionnel, dans la troisième direction (w), est pareil à l'étendue, corrigée des effets d'un opérateur de modulation et/ou d'un opérateur de démodulation de la première fonction (G.3D) partielle, de l'ensemble (x, x') tridimensionnel de données de mammographie dans la troisième direction (w).

8. Procédé suivant l'une des revendications précédentes, dans lequel l'ensemble (G_x, G_x') bidimensionnel de données de mammographie est étendu dans une première direction (u) et dans une deuxième direction (v), comprenant, en outre, le stade de procédé suivant :

- élimination (RMV) d'une couche (w.1, w.2, w.7, w.8) bidimensionnelle de l'ensemble (x) tridimensionnel de données de mammographie, au moyen de l'unité (DS.2) de calcul, la couche (w.1, w.2, w.7, w.8) bidimensionnelle étant étendue dans la première direction (u) et dans la deuxième direction (v).

9. Procédé suivant la revendication 8, dans lequel on détermine la couche (w.1, w.2, w.7, w.8) bidimensionnelle sur la base de la position des mamelons dans l'ensemble (x) tridimensionnel de données de mammographie.

10. Procédé suivant la revendication 8, dans lequel on détermine la couche (w.1, w.2, w.7, w.8) bidimensionnelle sur la base de facteurs de pondération de voxel de l'ensemble (x) tridimensionnel de données de mammographie, un facteur de pondération d'un voxel correspondant à la probabilité que le voxel représente une lésion.

**11.** Procédé suivant l'une des revendications précédentes, dans lequel l'ensemble (G_x, G_x') bidimensionnel de données de mammographie comprend un ou plusieurs pixels, comprenant, en outre, le stade de procédé suivant :

- deuxième détermination (DET-2) d'un ensemble (GPM_x, GPM_x') bidimensionnel de données de probabilité au moyen de l'unité (DS.2) de calcul,
l'ensemble (GPM_x, GPM, x') bidimensionnel de données de probabilité associant, à chacun du un ou des plusieurs pixels de l'ensemble (G_x, G_x') bidimensionnel de données de mammographie, une probabilité que le pixel respectif reproduise une lésion.

**12.** Système (DS) de détermination, comprenant les unités suivantes :

- interface (DS.1), constituée pour recevoir (REC) un ensemble (x, x') tridimensionnel de données de mammographie d'une partie à étudier,
- unité (DS.2) de calcul, constituée pour la première détermination (DET-1) d'un ensemble (G_x, G_x') bidimensionnel de données de mammographie de la partie à étudier, par application d'une fonction (G) de générateur ayant subi un apprentissage à l'ensemble (x, x') tridimensionnel de données de mammographie,
la fonction (G) de générateur ayant subi un apprentissage reposant sur un réseau GA (GA) ayant subi un apprentissage.

**13.** Système (DS) de détermination suivant la revendication 12, constitué, en outre, pour exécuter un procédé suivant les revendications 2 à 11.

**14.** Produit de programme d'ordinateur, ayant un programme d'ordinateur, qui peut être chargé directement dans une unité (DS.3) de mémoire d'un système (DS) de détermination, comprenant des parties de programme pour effectuer tous les stades du procédé suivant l'une des revendications 1 à 11, lorsque les parties de programme sont réalisées par le système (DS) de détermination.

**15.** Support de mémoire déchiffrable par ordinateur, sur lequel des parties de programme, pouvant être lues et réalisées par un système (DS) de détermination, sont mémorisées pour effectuer tous les stades du procédé suivant l'une des revendications 1 à 11, lorsque les parties de programme sont réalisées par le système (DS) de détermination.

FIG 1

REC

RMV

DET-1

DET-2

FIG 2

DS.1

DS.2

DS.3

DS.4

DS

FIG 3

FIG 4

FIG 5

X, X'     CL.3D     FV.3D     G     FV.2D     CL.2D     G_x, G_x'

G.3D     G.PL     G.2D

EP 3 462 412 B1

FIG 6

x, x'    CL.3D    FV.3D    G.PL    FV.2D    CL.2D    G_x, G_x'

G.3D    G.2D

GPM_x, GPM_x'

CL.2D    G.PM2D

G

EP 3 462 412 B1

FIG 7

FIG 8

**IN DER BESCHREIBUNG AUFGEFÜHRTE DOKUMENTE**

**In der Beschreibung aufgeführte Patentdokumente**

- US 20170011534 A1

**In der Beschreibung aufgeführte Nicht-Patentliteratur**

- **FELIX DIEKMANN et al.** Thick Slices from Tomosynthesis Data Sets: Phantom Study for the Evaluation of Different Algorithms. *Journal of Digital Imaging,* 2009, vol. 22 (5), 519-526

- **G. VAN SCHIE et al.** Mass detection in reconstructed digital breast tomosynthesis volumes with a computer-aided detection system trained on 2D mammograms. *Medical Physics,* 2013, vol. 40 (4), 041902
- **IAN J. GOODFELLOW.** *Generative Adversarial Networks,* 2014